(19) Europäisches Patentamt
European Patent Office
Office européen des brevets

(11) EP 1 721 008 B1

(12) **EUROPEAN PATENT SPECIFICATION**

(45) Date of publication and mention of the grant of the patent:
**24.02.2010 Bulletin 2010/08**

(21) Application number: **05716834.6**

(22) Date of filing: **28.02.2005**

(51) Int Cl.:
***C12Q 1/68*** *(2006.01)*

(86) International application number:
**PCT/EP2005/050850**

(87) International publication number:
**WO 2005/085472 (15.09.2005 Gazette 2005/37)**

(54) **DIAGNOSTIC AND THERAPEUTIC USE OF MAL2 GENE AND PROTEIN FOR NEURODEGENERATIVE DISEASES**

DIAGNOSTISCHE UND THERAPEUTISCHE VERWENDUNG DES MAL2-GENS UND PROTEINS BEI NEURODEGENERATIVEN KRANKHEITEN

UTILISATION DIAGNOSTIQUE ET THERAPEUTIQUE DU GENE ET DE LA PROTEINE MAL2 POUR LES MALADIES NEURODEGENERESCENTES

(84) Designated Contracting States:
**AT BE BG CH CY CZ DE DK EE ES FI FR GB GR HU IE IS IT LI LT LU MC NL PL PT RO SE SI SK TR**

(30) Priority: **03.03.2004 US 549147 P**

(43) Date of publication of application:
**15.11.2006 Bulletin 2006/46**

(73) Proprietor: **EVOTEC Neurosciences GmbH**
**22525 Hamburg (DE)**

(72) Inventors:
- **VON DER KAMMER, Heinz**
  **22607 Hamburg (DE)**
- **POHLNER, Johannes**
  **22175 Hamburg (DE)**

(74) Representative: **Meyers, Hans-Wilhelm et al**
**Patentanwälte**
**von Kreisler Selting Werner**
**Postfach 10 22 41**
**50462 Köln (DE)**

(56) References cited:
**WO-A-01/53343**

- **DATABASE Geneseq [Online] 8 August 2001 (2001-08-08), "Human gene 5 encoded secreted protein HETKL27, SEQ ID NO: 68." XP002341369 retrieved from EBI accession no. GSP:AAE03822 Database accession no. AAE03822 & WO 01/36440 A (HUMAN GENOME SCIENCES, INC; RUBEN, STEVEN, M; KOMATSOULIS, GEORGE, A;) 25 May 2001 (2001-05-25)**
- **DATABASE Geneseq [Online] 11 December 2002 (2002-12-11), "Human ovarian cancer marker M472." XP002341370 retrieved from EBI accession no. GSP:ABG96412 Database accession no. ABG96412 & WO 02/071928 A (MILLENNIUM PHARMACEUTICALS, INC; MONAHAN, JOHN, E; GANNAVARAPU, MANJUL) 19 September 2002 (2002-09-19)**
- **DATABASE Geneseq [Online] 27 August 2002 (2002-08-27), "Human albumin fusion protein #1240." XP002341371 retrieved from EBI accession no. GSP:ABG64565 Database accession no. ABG64565 & WO 01/77137 A (HUMAN GENOME SCIENCES, INC; ROSEN, CRAIG, A; HASELTINE, WILLIAM, A) 18 October 2001 (2001-10-18)**
- **DATABASE Geneseq [Online] 20 January 2003 (2003-01-20), "Human polypeptide SEQ ID NO 1722." XP002341372 retrieved from EBI accession no. GSP:ABP69675 Database accession no. ABP69675 & WO 02/070539 A (HYSEQ, INC; TANG, Y., TOM; ZHOU, PING; GOODRICH, RYLE, W; ASUNDI, VINO) 12 September 2002 (2002-09-12)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MARAZUELA MONICA ET AL: "Expression and distribution of MAL2, an essential element of the machinery for basolateral-to-apical transcytosis, in human thyroid epithelial cells." ENDOCRINOLOGY, vol. 145, no. 2, February 2004 (2004-02), pages 1011-1016, XP002340879 ISSN: 0013-7227
- WILSON S H D ET AL: "Identification of MAL2, a Novel Member of the MAL Proteolipid Family, Though Interactions with TPD52-like Proteins in the Yeast Two-Hybrid System" GENOMICS, ACADEMIC PRESS, SAN DIEGO, US, vol. 76, no. 1-3, August 2001 (2001-08), pages 81-88, XP004459581 ISSN: 0888-7543
- ECKELMAN WILLIAM C: "The use of PET and knockout mice in the drug discovery process." DRUG DISCOVERY TODAY. 1 MAY 2003, vol. 8, no. 9, 1 May 2003 (2003-05-01), pages 404-410, XP002340880 ISSN: 1359-6446
- HUSE J T ET AL: "Neurotoxic traffic: uncovering the mechanics of amyloid production in Alzheimer’s disease." TRAFFIC (COPENHAGEN, DENMARK) FEB 2001, vol. 2, no. 2, February 2001 (2001-02), pages 75-81, XP002340881 ISSN: 1398-9219

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β protein evolves from the cleavage of the amyloid precursor protein (APP) by different kinds of proteases. The cleavage by the ß/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). They are primarily found in the cerebral cortex and hippocampus. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. The appearance of neurofibrillary tangles and their increasing number correlates well with the clinical severity of AD (Schmitt et al., Neurology 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). The age of onset of AD may vary within a range of 50 years, with early-onset AD occurring in people younger than 65 years of age, and late-onset of AD occurring in those older than 65 years.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

Myelin and lymphocyte proteolipid (MAL) is the founder member of a synonymous family of proteins with structural and biochemical similarities (Perez et al., Biochem Biophys Res Commun 1997, 232:618-621). MAL is a nonglycosylated integral membrane protein that exclusively resides in rafts, containing four hydrophobic transmembrane (TM1-4) segments, with cytoplasmic N- and C-termini, forming a so-called "MARVEL" domain (MAL and Related proteins for Vesicle trafficking and membrane Link), a conserved domain involved in membrane apposition events. MAL plays an essential role in the direct-route transport of proteins with apical destination. As an itinerant protein, MAL cyclically shuttles between the trans-Golgi network, the plasma membrane and the endosomes. MAL is required for the correct delivery of apical cargo (both membrane bound and secretory proteins) within polarized cells (e. g. epithelia, oligodendrocytes, Schwann cells), and it is a key element in the formation, sorting and transport of vesicles, and in cholesterol-rich membrane apposition events, including the organization and maintenance of membrane microdomains/lipid rafts (Puertollano and Alonso, Mol Biol Cell 1999, 10:3435-3447; Martin-Belmonte et al., J Biol Chem 2001, 276:49337-49342; Sanchez-Pulido et al., Trends Biochem Sci 2002, 27:599-601; Erne et al., J Neurochem 2002, 82:550-562).
Not only epithelial cells, but also myelin forming glia cells in the central (CNS) and peripheral nervous system (PNS), oligodendrocytes and Schwann cells, are polarized cells with distinct transport and sorting pathways. A proposed model of sphingolipid-cholesterol raft dependent organization and control of transport and sorting may therefore also apply to myelin. MAL is expressed by oligodendrocytes and Schwann cells as a component of central and peripheral myelin, where it is selectively enriched, together with CD59, in detergent-insoluble glycolipid enriched membrane microdomains (DIGs). In the CNS, MAL is involved in late steps of myelin sheath formation and myelin compaction, whereas in the PNS it plays a role in the terminal differentiation of maturating Schwann cells and in the onset of myelination (Erne et al., J Neurochem 2002, 82:550-562; Frank et al., J Neurochem 2000, 75:1927-1939; Frank et al., J Neurochem 1999, 73:587-597).
MAL2 has recently been identified as a novel member of the MAL family, sharing 36% sequence identity with MAL at the protein level (Wilson et al., Genomics 2001, 76:81-88; Genbank data base accession numbers: genomic DNA contig AC009514, cDNA AY007723, protein Q969L2). The MAL2 gene maps to chromosome 8q24.12 and it comprises 4 exons. Like MAL, MAL2 is an integral membrane protein whose structure is based on the characteristic 4 transmembrane-helix MARVEL domain with cytoplasmic N- and C-termini. It also contains the characteristic MAL-like sequence motif (Q/F)GWVM(F/Y)V in TM2 (Gln65-Val71). Furthermore, the C-terminal LRRW sequence motif of MAL2 (aa 171-174) is similar to the MAL motif LIRW (aa 146-149), which has been shown to be a minimal requirement for the targeting to glycolipid enriched membrane microdomains. Despite these similarities, MAL2 differs from MAL and other MAL-like proteins in some other aspects: it has a longer N-terminal domain 34n instead of 15-22 aa) with a higher proline content (29% versus 7-19%) and an FPPP sequence (aa 18-21) resembling an FPPPP recognition motif for EVH1 (enabled, VASP, homology 1) domains; and it possesses an additional 10-aa insertion (Ser125-Thr124) predicting a larger loop of 31-aa between TM3 and TM4 (luminal), with a single N-glycosylation site (Asn132). Whereas MAL is not glycosylated, Western blot analysis of MAL2 revealed a distinct band at 19 kDa and an endogycosylase H sensitive smear at ~30-40 kDa, reflecting both unglycosylated and glycosylated MAL2, respectively, indicating that MAL2 is partly N-glycosylated (Wilson et al., Genomics 2001, 76:81-88; DeMarco et al., J Cell Biol 2002, 159:37-44; Sanchez-Pulido et al., Trends Biochem Sci 2002, 27:599-601; Magyar et al., Gene 1997, 189:269-275). Northern blot analyses of human tissue mRNA extracts revealed a major 2.8 kb MAL2 transcript with highest signal intensity in the kidney and in mammary carcinoma, moderate levels in brain, liver and lung, weak expression in heart, placenta, colon (without mucosa) and small intestine. A minor 1.2 kb transcript produced a moderate signal in mammary carcinoma and weak signals in kidney and liver. An endogenous 2.8 kb transcript was detected in HepG2, Caco-2 (both human) and MDCK (canine) cell lines, but not in Jurkat, HPB-ALL, A498, HeLa and K-562 (all human) or COS-7 (simian) cells. The 1.2 kb signal has been proposed to reflect the existence of a 3'-UTR truncated MAL2 transcript due to an alternative (earlier) polyadenylation (AATAAA) signal within the 3'-UTR (nt 1190-1195 of AY007723) (DeMarco et al., J Cell Biol 2002, 159:37-44; Wilson et al., Genomics 2001, 76:81-88). DeMarco et al. (J Cell Biol 2002, 159:37-44) have shown that antisense oligonucleotide mediated depletion of endogenous MAL2 drastically blocked apically targeted transport of both exogenous and endogenous transcytosing molecules at perinuclear endosomes. MAL2 depletion did not affect the internalization of these molecules but caused their accumulation in perinuclear endosome elements that were accessible to transferrin. From their data DeMarco et al. conclude that both MAL and MAL2 are essential and functionally distinct members of the machinery of polarized transport, in that MAL plays a key role in the direct apical transport pathway, and MAL2 is required for the indirect transcytotic route.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A derivative translation product, for instance, may be generated by processes such as altered phosphorylation, or glycosylation, or acetylation, or lipidation, or by altered signal peptide cleavage or other types of maturation cleavage. These processes may occur post-translationally. Preferably, a "modulator" is capable of changing or altering the biological activity of a transcription product or a translation product of a gene. Said modulation, for instance, may be an increase or a decrease in the biological activity and/or pharmacological activity, in enzyme activity, a change in binding characteristics, or any other change or alteration in the biological, functional, or immunological properties of said translation product of a gene. A "modulator" refers to a molecule which has the capacity to either enhance or inhibit, thus to "modulate" a functional property of an ion channel subunit or an ion channel, to "modulate" binding, antagonization, repression, blocking, neutralization or sequestration of an ion channel or ion channel subunit and to "modulate" activation, agonization and upregulation. "Modulation" will be also used to refer to the capacity to affect the biological activity of a cell. The terms "modulator", "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. They may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. Such modulators, agents, reagents or compounds can be factors present in cell culture media, or sera used for cell culturing, factors such as trophic factors. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same ty pe (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLA STN 2.0 (Gish W., http://blast.wustl.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453). The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention, but retains its essential properties. Furthermore, the term "variant" shall include any shorter or longer version of a pol ypeptide or protein. "Variants" shall also comprise a sequence that has at least a bout 80% sequence identity, more preferably at least about 90% sequence identity, and most preferably at least about 95% sequence identity with the amino acid sequences of MAL2 protein, SEQ ID NO: 1. "Variants" include, for example, proteins with conservative amino acid substitutions in highly conservative regions. "Proteins and polypeptides" of the present invention include variants, fragments and chemical derivatives of the protein comprising the amino acid sequences of MAL2 protein, SEQ ID NO: 1. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature, it is also said that they are "non-native".

This notion further means that the sequences encoding such molecules can be linked by the hand of man to polynucleotides to which they are not linked in their natural state and such molecules can be produced by recombinant and/or synthetic means (non-native). Even if for said purposes those sequences may be introduced into living or non-living organisms by methods known to those skilled in the art, and even if those sequences are still present in said organisms, they are still considered to be isolated, to be non-native. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christemt Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998). The term "Braak stage" or "Braak staging" refers to the classification of brains according to the criteria proposed by Braak and Braak (Braak and Braak, Acta Neuropathology 1991, 82: 239-259). On the basis of the distribution of neurofibrillary tangles and neuropil threads, the neuropathologic progression of AD is divided into six stages (stage 0 to 6). In the instant invention Braak stages 0 to 2 represent healthy control persons ("controls"), and Braak stages 4 to 6 represent persons suffering from Alzheimer's disease ("AD patients"). The values obtained from said "controls" are the "reference values" representing a "known health status" and the values obtained from said "AD patients" are the "reference values" representing a "known disease status". Braak stage 3 (middle Braak stage) may represent either a healthy control persons or an AD patient. The higher the Braak stage the more likely is the possibility to display the symptoms of AD. For a neuropathological assessment, i.e. an estimation of the probability that pathological changes of AD are the underlying cause of dementia, a recommendation is given by Braak H. (www. alzforum.org).

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

The present invention discloses the detection, identification and the differential regulation, a dysregulation of a gene of the Myelin and lymphocyte proteolipid (MAL) gene family coding for a member of the MAL family of proteins, the Myelin and lymphocyte proteolipid (MAL) protein MAL2, in samples of specific brain regions of Alzheimer's disease patients in comparison to the respective samples of age-matched control persons. The present invention discloses that the gene expression for MAL2 is varied, is dysregulated within different regions of AD-affected brains, in that MAL2 mRNA levels are lowered, are down-regulated in the temporal cortex and/or the hippocampus as compared to the frontal cortex, or are upregulated in the frontal cortex as compared to the temporal cortex and/or the hippocampus. Further, the present invention discloses that the MAL2 expression differs between the frontal cortex and the temporal cortex and/or the hippocampus of healthy age-matched control subjects compared to the frontal cortex and the temporal cortex and/or the hippocampus of AD patients. No such dysregulation is observed within samples of different brain regions obtained from age-matched, healthy controls. MAL2 is lowered in the temporal cortex and in the frontal cortex of AD-patients compared to the temporal cortex and frontal cortex of controls. This dysregulation presumably relates to a pathologic alteration of MAL2 signaling in AD-affected brains. To date, no experiments have been described that demonstrate a relationship between the dysregulation of MAL2 gene expression and the pathology of neurodegenerative diseases, in particular AD. Likewise, no mutations in the MAL2 gene have been described to be associated with said diseases. Linking the MAL2 gene to such diseases offers new ways, inter alia, for the diagnosis and treatment of said diseases. The present invention discloses a dysregulation of a gene coding for MAL2 in specific brain regions of AD patients. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Therefor, brain tissues from the frontal cortex (F), the temporal cortex (T), and the hippocampus (H) of AD patients and of healthy, age-matched control individuals, respectively, were used for the herein disclosed examples. Consequently, the MAL2 gene and its corresponding transcription and/or translation products have a causative role in the regional selective neuronal degeneration typically observed in AD. Alternatively, the gene coding for MAL2 protein and its products may confer neuroprotective functions to nerve cells. Based on these disclosures, the present invention has utility for the diagnostic evaluation and prognosis as well as for the identification of a predisposition to AD. Furthermore, the present invention provides methods for the diagnostic monitoring of patients undergoing treatment for such a disease.

In one aspect, the invention features a method of diagnosing or prognosticating Alzheimer's disease in a subject, or determining whether a subject is at increased risk of developing said disease. The method comprises: determining a level, or an activity, or both said level and said activity of (i) a transcription product of the gene coding for MAL2 protein, and/or of (ii) a translation product of the gene coding for MAL2 protein in a sample obtained from said subject and comparing said level, and/or said activity of said transcription product and/or said translation product to a reference value representing a known disease status and/or to a reference value representing a known health status (healthy control), and said level and/or said activity is varied, is altered compared to a reference value representing a known health status, and/or is similar or equal to a reference value representing a known disease status, thereby diagnosing or prognosticating said neurodegenerative disease in said subject, or determining whether said subject is at increased risk of developing said neurodegenerative disease. The wording "in a subject" refers to results of the methods disclosed as far as they relate to a disease afflicting a subject, that is to say, said disease being "in" a subject.

The invention also discloses the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further discloses the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit. Primers for MAL2 are exemplarily described in Example (iv).

The invention discloses a method of monitoring the progression of a neurodegenerative disease in a subject. A level, or an activity, or both said level and said activity, of (i) a transcription product of the gene coding for MAL2 protein, and/or of (ii) a translation product of the gene coding for MAL2 protein in a sample from said subject is determined. Said level and/or said activity is compared to a reference value representing a known disease or health status. Thereby, the progression of said neurodegenerative disease in said subject is monitored.

Further, the invention discloses a method of evaluating a treatment for a neurodegenerative disease, comprising determining a level, or an activity, or both said level and said activity of (i) a transcription product of the gene coding for MAL2 protein, and/or of (ii) a translation product of the gene coding for MAL2 protein in a sample obtained from a subject being treated for said disease. Said level, or said activity, or both said level and said activity are compared to a reference value representing a known disease or health status, thereby evaluating the treatment for said neurodegenerative disease.

According to the instant invention, said Myelin and lymphocyte proteolipid (MAL) gene and protein, is a member of the MAL family of proteins, is the Myelin and lymphocyte proteolipid (MAL) gene and protein MAL2. MAL2 is represented by the gene coding for the protein of the SwissProt Genbank accession number Q969L2. The amino acid sequence of said protein is deduced from the mRNA seqeunce corresponding to the cDNA sequence of Genbank accession number AY007723 and of the genomic DNA contig AC009514. In the instant invention MAL2 also refers to the nucleic acid sequence of SEQ ID NO: 2, coding for the protein of SEQ ID NO: 1 (Genbank accession number Q969L2) and to SEQ ID NO:4 which corresponds to the coding sequence of MAL2 (MAL2cds). In the instant invention said sequences are "isolated" as the term is employed herein. Further, in the instant invention, the gene coding for said MAL2 protein is also generally referred to as the MAL2 gene, or simply MaL2, and the protein of MAL2 is also generally referred to as the MAL2 protein, or simply MAL2.

It is preferred that the sample to be analyzed and determined is selected from the group comprising brain tissue or other tissues, or body cells. The sample can also comprise cerebrospinal fluid or other body fluids including saliva, urine, Alzheimer's blood, serum plasma, or mucus. Preferably, the methods of diagnosis, prognosis disease, according to the instant invention, can be practiced ex *corpore*, and such methods preferably relate to samples, for instance, body fluids or cells, removed, collected, or isolated from a subject or patient or healthy control person.

In further preferred embodiments, said reference value is that of a level, or an activity, or both said level and said activity of (i) a transcription product of the gene coding for MAL2 protein, and/or of (ii) a translation product of the gene coding for MAL2 protein in a sample obtained from a subject not suffering from said neurodegenerative disease (healthy control person, control sample, control) or in a sample obtained from a subject suffering from a neurodegenerative disease, in particular Alzheimer's disease (patient sample, patient).

An alteration in the level and/or activity of a transcription product of the gene coding for MAL2 protein and/or of a translation product of the gene coding for MAL2 protein in a sample cell, or tissue, or body fluid obtained from said subject relative to a reference value representing a known health status (control sample) indicates a diagnosis, or prognosis, or increased risk of becoming diseased AD.

An equal or similar level and/or activity of a transcription product of the gene coding for a MAL2 protein and/or of a translation product of the gene coding for a MAL2 protein in a sample cell, or tissue, our body fluid obtained from a subject relative to a reference value representing a known disease status of Alzheimer's disease (AD patient sample), indicates a diagnosis, or prognosis, or increased risk of becoming diseased with said neurodegenerative disease.

In preferred embodiments, measurement of the level of transcription products of the gene coding for MAL2 protein is performed in a sample obtained from a subject using a quantitative PCR-analysis with primer combinations to amplify said gene specific sequences from cDNA obtained by reverse transcription of RNA extracted from a sample of a subject. A Northern blot with probes specific for said gene can also be applied. It might be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of the gene coding for MAL2 protein and/or the level of activity of said translation product can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs. Western blots and other techniques known to those of ordinary skill in the art (see Harlow and Lane, Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In a preferred embodiment, the level, or the activity, or both said level and said activity of (i) a transcription product of the gene coding for MAL2 protein, and/or of (ii) a translation product of the gene coding MAL2 protein in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

The invention discloses a method of treating or preventing a neurodegenerative disease, in particular AD, in a subject comprising the administration to said subject in a therapeutically or prophylactically effective amount of an agent or agents which directly or indirectly affect a level, or an activity, or both said level and said activity, of (i) the gene coding for MAL2 protein, and/or (ii) a transcription product of the gene coding for MAL2 protein, and/or (iii) a translation product of the gene coding for MAL2 protein, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of the gene coding for MAL2 protein, or a fragment, or derivative, or a variant thereof. An agent for treating or preventing a neurodegenerative disease, in particular AD, according to the instant invention, may also consist of a nucleotide, an oligonucleotide, or a polynucleotide. Said oligonucleotide or polynucleotide may comprise a nucleotide sequence of the gene coding for MAL2 protein, either in sense orientation or in antisense orientation.

The method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

The invention discloses a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). The subject to be treated is a human, and therapeutic antisense nucleic acids or derivatives thereof are directed against transcription products of the gene coding for MAL2 protein. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skilled in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA Interference (RNAi), can be another effective approach for nucleic acid therapy (Hannon, Nature 2002, 418: 244-251).

Further the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see McCelland and Pardee, Expression Genetics: Accelerated and High-Throughput Methods, Eaton - Publishing, Natick, MA, 1999).

Said agent for treating and preventing AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In a further aspect, the invention features the use of a recombinant, non-human animal comprising a non-native MAL2 gene sequence as an animal model for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of a neurodegenerative disease, in particular AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). It is preferred to make use of such a recombinant non-human animal.

In another aspect, the invention features an assay for screening for a modulator of neurodegenerative diseases, in particular Alzheimer's disease, or related diseases or disorders of one or more substances selected from the group consisting of

(i) a gene coding for MAL2 protein, and/or
(ii) a transcription product of the gene coding for MAL2 protein, and/or
(iii) a translation product of the gene coding for MAL2 protein, said method comprising:

(a) contacting a cell with a test compound;
(b) measuring the activity and/or level of one or more substances recited in (i) to (iii);
(c) measuring the activity and/or level of one or more substances recited in (i) to (iii) in a control cell not contacted with said test compound; and comparing the levels and/or activities of the substance in the cells of step (b) and (c), wherein an alteration in the activity and/or level of substances in the contacted cells indicates that the test compound is a modulator of said diseases or disorders.

In one further aspect, the invention features a method of screening for a modulator of neurodegenerative diseases, in particular Alzheimer's disease, or related diseases or disorders of one or more substances selected from the group consisting of

(i) the gene coding for MAL2 protein, and/or
(ii) a transcription product of the gene coding for MAL2 protein, and/or
(iii) a translation product of the gene coding for MAL2 protein,
said method comprising:

(a) administering a test compound to a non-human test animal which is predisposed to developing or has already developed symptoms of a neurodegenerative disease or related diseases or disorders in respect of the substances recited in (i) to (iii);
(b) measuring the activity and/or level of one or more substances recited in (i) to (iii);
(c) measuring the activity and/or level of one or more substances recited in (i) or (iii) in a matched non- human control animal which is predisposed to developing or has already developed symptoms of a neurodegenerative disease or related diseases or disorders in respect to the substances recited in (i) to (iii) and to which non-human animal no such test compound has been administered;
(d) comparing the activity and/or level of the substance in the non-human animals of step (b) and (c), wherein an alteration in the activity and/or level of substances in the non-human test animal indicates that the test compound is a modulator of said diseases or disorders.

In a preferred embodiment, said non-human test animal and/or said non-human control animal is a recombinant, non-human animal which expresses the gene coding for MAL2 protein under the control of a transcriptional regulatory element which is not the native MAL2 protein gene transcriptional control regulatory element.

The method of the invention is useful for an assay for testing a compound, preferably for screening a plurality of compounds, for inhibition of binding between a ligand and MAL2 protein. Said screening assay comprises the steps of (i) adding a liquid suspension of said MAL2 protein to a plurality of containers, and (ii) adding a compound or a plurality of compounds to be screened for said inhibition to said plurality of containers, and (iii) adding a detectable, preferably a fluorescently labelled ligand to said containers, and (iv) incubating said MAL2 protein and said compound or plurality of compounds, and said detectable, preferably fluorescently labelled ligand, and (v) measuring the amounts of preferably the fluorescence associated with said MAL2 protein and (vi) determining the degree of inhibition by one or more of said compounds of binding of said ligand to said MAL2 protein. It might be preferred to reconstitute said MAL2 translation product into artificial liposomes to generate the corresponding proteoliposomes to determine the inhibition of binding between a ligand and said MAL2 translation product. Methods of reconstitution of MAL2 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for MAL2 protein. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436 and WO 01/59416.

In one further embodiment, the present invention provides a method for producing a medicament comprising the steps of (i) identifying a compound as an inhibitor of binding between a ligand and a gene product of the gene coding for MAL2 protein by the aforementioned inhibitory binding assay and (ii) admixing the compound with a pharmaceutical carrier. However, said compound may also be identifiable by other types of screening assays.

The present invention features the use of a protein molecule and the use of said protein molecule as shown in SEQ ID NO:1, said protein molecule being a translation product of the gene coding for MAL2 as a diagnostic target for detecting Alzheimer's disease.

The present invention further features a protein molecule and the use of said protein molecule as shown in SEQ ID NO:1, said protein molecule being a translation product of the gene coding for MAL2 as a screening target for reagents or compounds preventing, or treating, or ameliorating Alzheimer's disease.

The present invention features the use of an antibody which is specifically immunoreactive with an immunogen, wherein said immunogen is a translation product of the gene coding for MAL2 protein, SEQ ID NO:1 for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to Alzheimer's disease.

The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodlagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the MAL2 gene.

Said antibodies can be used for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell. The pathological state relates to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

FIGURES:

Figure 1 discloses the initial identification of the differential expression of the gene coding for MAL2 protein in a fluorescence differential display screen. The figure shows a clipping of a large preparative fluorescent differential display gel. PCR products from the frontal cortex (F) and the temporal cortex (T) of two healthy control subjects and six AD patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the migration position where significant differences in intensity of the signals for a transcription product of the gene coding for MAL2 protein derived from frontal cortex and from the temporal cortex of AD patients as compared to healthy controls exist. The differential expression reflects a down-regulation of MAL2 gene transcription in the temporal cortices of AD patients compared to the temporal cortices of control persons.

Figure 2 and Figure 3 illustrate the verification of the differential expression of the MAL2 gene in AD brain tissues by quantitative RT-PCR analysis. Quantification of RT-PCR products from RNA samples collected from the frontal cortex (F) and the temporal cortex (T) of AD patients (Figure 2A) and samples from the frontal cortex (F) and the hippocampus (H) of AD patients (Figure 3A) was performed by the LightCycler rapid thermal cycling technique. Likewise, samples of healthy, age-matched control individuals were compared (Figure 2B for frontal cortex and temporal cortex, Figure 3B for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of MAL2 cDNAs from both, the frontal and temporal cortices of a normal control individual, and from the frontal cortex and hippocampus of a normal control individual, respectively, during the exponential phase of the reaction are juxtaposed (Figures 2B and 3B, arrowhead), whereas in Alzheimer's disease (Figures 2A and 3A, arrowhead) there is a significant separation of the corresponding curves, indicating a differential expression of the gene coding for MAL2 in the respective analyzed brain regions. The differential expression reflects a dysregulation, preferably a down-regulation of a transcription product of the human MAL2 gene, or a fragment, or derivative, or variant thereof, in the temporal cortex relative to the frontal cortex.

Figure 4 illustrates the verification of the differential expression of the human MAL2 gene in AD brain tissues (P) versus healthy control brain tissue samples (C) by quantitative RT-PCR analysis. Quantification of RT-PCR products from RNA samples collected from the frontal cortex region of AD patients and of healthy, age-matched control persons ($P_{(F)}$ - $C_{(F)}$; Figure 5A) was performed by the LightCycler rapid thermal cycling technique. Likewise, samples from the temporal cortex region of AD patients and of control individuals ($P_{(T)}$ - $C_{(T)}$; Figure 5B) were compared. The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. The curves delineating the amplification kinetics of MAL2 cDNAs are significantly separated during the exponential phase of the amplification reaction, for both brain regions analyzed: (i) frontal cortex of a normal control individual in comparison to frontal cortex of an AD patient (Figure 5A), and (ii) temporal cortex of a normal control individual in comparison to temporal cortex of an AD patient (Figure 5B). This indicates a differential expression of the gene coding for MAL2 in the analyzed brain regions of AD patients in comparison with healthy control persons and reflects a down-regulation of a transcription product of the human MAL2 gene, or a fragment, or derivative, or variant thereof, in the temporal cortex and in the frontal cortex of AD patients relative to the temporal cortex and the frontal cortex of healthy control persons.

Figure 5 discloses SEQ ID NO: 1, the amino acid sequence of the human MAL2 protein. The full length human MAL2 protein comprises 176 amino acids (aa), as defined by the SwissProt accession number Q969L2.

Figure 6 shows SEQ ID NO: 2, the nucleotide sequence of the human MAL2 cDNA, comprising 2808 nucleotides (nt), as defined by the Genbank accession number AY007723.

Figure 7 depicts SEQ ID NO: 3, the nucleotide sequence of the 270 bp MAL2 cDNA fragment, identified and obtained by differential display and subsequent cloning (sequence in 5' to 3' direction).

Figure 8 shows the nucleotide sequence of SEQ ID NO: 4, the coding sequence (cds) of the human MAL2 gene, comprising 531 nucleotides (nucleotides 80-610 of SEQ ID NO: 2).

Figure 9 outlines the sequence alignment of SEQ ID NO: 3 to the nucleotide sequence of MAL2 cDNA (SEQ ID NO: 2).

Figure 10 lists MAL2 gene expression levels in the temporal cortex relative to the frontal cortex in fifteen AD patients, herein identified by internal reference numbers P010, P011, P012, P014, P016, P017, P019, P038, P040, P041, P042, P046, P047, P048, P049 (1.02 to 3.45 fold, values according to the formula described below) and twentyfive healthy, age-matched control individuals, herein identified by internal reference numbers C005, C008, C011, C012, C014, C025, C026, C027, C028, C029, C030, C031, C032, C033, C034, C035, C036, C038, C039, C041, C042, DE02, DE03, DE05, DE07 (0.29 to 3.70 fold, values according to the formula described below). For an up-regulation in the tem poral cortex, the values shown are calculated according to the formula described herein (see below) and in case of an up-regulation in the frontal cortex the reciprocal values of the formula described herein are calculated, respectively. The bar diagram visualizes individual natural logarithmic values of the tem poral to frontal cortex, ln(IT/IF), and of the frontal to temporal cortex regulation factors, ln(IF/IT), in different Braak stages (0 to 6). An obvious difference reflecting a down-regulation in the temporal cortex is shown. The Braak stages correlate with the progressive course of AD disease which, as shown in the instant invention, is associated with an increasing difference in the regulation, the level and the activity of MAL2 as described above.

Figure 11 lists the gene expression levels in the hippocampus relative to the frontal cortex for the MAL2 gene in six Alzheimer's disease patients, herein identified by internal reference numbers P010, P011, P012, P014, P016, P019 (0.12 to 2.04 fold) and three healthy, age-matched control individuals, herein identified by internal reference numbers C004, C005, C008 (0.62 to 1.00 fold). The values shown are calculated according to the formula described herein (see below). The scatter diagram visualizes individual logarithmic values of the hippocampus to frontal cortex regulation ratios, log(ratio HC/IF), in control samples (dots) and in AD patient samples (triangles).

Figure 12 shows the analysis of absolute mRNA expression of MAL2 (alias ens0711) by comparison of control and AD stages using statistical method of the median at 98%-confidence level. The data were calculated by defining control groups including subjects with either Braak stages 0 to 1, Braak stages 0 to 2, or Braak stages 0 to 3 which are compared with the data calculated for the defined AD patient groups including Braak stages 2 to 6, Braak stages 3 to 6 and Braak stages 4 to 6, respectively. Additionally, three groups including subjects with either Braak stages 0 to 1, Braak stages 2 to 3 and Braak stages 4 to 6, respectively, were compared with each other. A difference was detected comparing frontal cortex (F) and inferior temporal cortex (T) of AD patients and of healthy age-matched control persons with each other. Said difference reflects a down-regulation of MAL2 in the temporal cortex and in the frontal cortex of AD patients relative to the temporal cortex and frontal cortex of healthy age-matched control persons.

Figure 13 depicts a Western blot image of total cell protein extracts labeled with polyclonal anti-myc antibody (MBL, 1:1000).

Lanes A and B: total protein extract of H4APPsw cells stably expressing MAL2 tagged with a myc-tag (MAL2-myc, A) and control H4APPsw cells (B). The arrow indicates a major band at about 19 kDa (lane A), which corresponds to the predicted molecular weight of the MAL2 protein.

Figure 14 shows the immunofluorescence analysis of H4APPsw control cells and H4APPsw cells stably over-expressing the myc-tagged MAL protein (H4APPsw-MAL2 cds-myc). The MAL2-myc protein was detected with rabbit polyclonal anti-myc antibodies (MBL) and a Cy3-conjugated anti-rabbit antibody (Amersham) (Figures 14A and 14B).

The cellular nucleus was stained with DAPI (Figures 14C and 14D). The overlay analysis indicate that the MAL2-myc protein is mainly localized to the golgi and the plasma membrane (Figure 14E) and is over-expressed in more than 70% of the H4APPsw-MAL2cds-myc transduced cells as compared to the H4APPsw control cells (Figure 14F).

Figure 15 depicts sections from human frontal cortex labeled with an affinity-purified rabbit polyclonal anti-MAL2 antibodies (green signals) raised against a peptide corresponding to amino acids 22-38 of MAL2. Immunoreactivity of MAL2 was observed in both the cerebral cortex (CT) and the white matter (WM) (Figure 15A, low magnification). MAL2 immunoreactivity was observed mainly in the cytoplasm and also in plasma membranes of neurons and glial cells in the cortex (Figure 15B, high magnification). Figure 15C shows MAL2 colocalization in the white matter with CNPase (2',3'-cyclic nucleotide 3'-phosphodiesterase, red signals) in oligodendrocytic cell bodies (indicated by arrows) and to a lesser extent in myelin sheets. Blue signals indicate nuclei stained with DAPI.

EXAMPLE I:

(i) Brain tissue dissection from patients with AD:

Brain tissues from AD patients and healthy, age-matched control subjects were collected, on average, within 6 hours post-mortem and immediately frozen on dry ice. Sample sections from each tissue were fixed in paraformaldehyde for histopathological confirmation of the diagnosis. Brain areas for differential expression analysis were identified and stored at -80 °C until RNA extractions were performed. Tissues of several brain regions, the frontal cortex (F), the temporal cortex (T) and the hippocampus (H), which exhibit selective vulnerability to neuronal loss and degeneration in AD, were used for the herein disclosed examples.

(ii) Isolation of total mRNA:

Total RNA was extracted from post-mortem brain tissue by using the RNeasy kit (Qiagen) according to the manufacturer's protocol. The accurate RNA concentration and the RNA quality were determin ed with the DNA LabChip system using the Agilent 2100 Bioanalyzer (A-gilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler tech nology as described in the supplied protocol by the manufacturer (Roche).

(iii) cDNA synthesis and identification of differentially expressed genes by fluorescence differential display (FDD):

In order to identify changes in gene expression in different tissue, a modified and improved differential display (DD) screening method was employed. The original DD screening method is known to those skilled in the art (Liang and Pardee, Science 1995, 267:1186-7). This technique compares two populations of RNA and provides clones of genes that are expressed in one population but not in the other. Several samples can be analyzed simultaneously and both up-and down-regulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers were developed (standard set) to achieve a statistically comprehensive analysis of all possible RNA species. Further, the method was modified to generate a preparative DD slab-gel technique, based on the use of fluorescently labelled primers. In the present invention, RNA populations from carefully selected post-mortem brain tissues (frontal and temporal cortex) of Alzheimer's disease patients and age-matched control subjects were compared.

As starting material for the DD analysis we used total RNA, extracted as described above (ii). Equal amounts of 0.05 μg RNA each were transcribed into cDNA in 20 μl reactions containing 0.5 mM each dNTP, 1 μl Sensiscript Reverse Transcriptase and 1x RT buffer (Qiagen), 10 U RNase inhibitor (Qiagen) and 1 μM of either one-base-anchor oligonucleotides $HT_{11}A$, $HT_{11}G$ or $HT_{11}C$ (Liang et al., Nucleic Acids Research 1994, 22: 5763-5764; Zhao et al., Biotechniques 1995, 18: 842-850). Reverse transcription was performed for 60 min at 37°C with a final denaturation step at 93°C for 5 min. 2 μl of the obtained cDNA each was subjected to a polymerase chain reaction (PCR) employing the corresponding one-base-anchor oligonucleotide (1 μM) along with either one of the Cy3 labelled random DD primers (1 μM), 1x GeneAmp PCR buffer (Applied Biosystems), 1.5 mM $MgCl_2$ (Applied Biosystems), 2 μM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 μl final volume. PCR conditions were set as follows: one round at 94°C for 30 sec for denaturing, cooling 1°C/sec down to 40°C, 40°C for 4 min for low-stringency annealing of primer, heating 1°C/sec up to 72°C, 72°C for 1 min for extension. This round was followed by 39 high-stringency cycles: 94°C for 30 sec, cooling 1°C/sec down to 60°C, 60°C for 2 min, heating 1°C/sec up to 72°C, 72°C for 1 min. One final step at 72°C for 5 min was added to the last cycle (PCR cycler: Multi Cycler PTC 200, MJ Research). 8 μl DNA loading buffer were added to the 20 μl PCR product preparation, denatured for 5 min and kept on ice until loading onto a gel. 3.5 μl each were separated on 0.4 mm thick, 6% polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, differentially expressed bands marked, excised from the gel, transferred into 1.5 ml containers, overlayed with 200 μl sterile water and kept at -20°C until extraction.

Elution and reamplification of DD products: The differential bands were extracted from the gel by boiling in 200 μl $H_2O$ for 10 min, cooling down on ice and precipitation from the supernatant fluids by using ethanol (Merck) and glycogen/ sodium acetate (Merck) at -20°C over night, and subsequent centrifugation at 13.000 rpm for 25 min at 4°C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the DD PCR (see above) under identical conditions, with the exception of the initial round at 94°C for 5 min, followed by 15 cycles of: 94°C for 45 sec, 60°C for 45 sec, ramp 1°C/sec to 70°C for 45 sec, and one final step at 72°C for 5 min.

Cloning and sequencing of DD products: Re-amplified cDNAs were analyzed with the DNA LabChip system (Agilent 2100 Bioanalyzer, Agilent Technologies) and ligated into the pCR-Blunt II-TOPO vector and transformed into *E.coli* Top10F' cells (Zero Blunt TOPO PCR Cloning Kit, Invitrogen) according to the manufacturer's instructions. Cloned cDNA fragments were sequenced by commercially available sequencing facilities. The result of one such FDD experiment for the gene coding for MAL2 protein is shown in Figure 1.

(iv) Confirmation of differential expression by quantitative RT-PCR:

Positive corroboration of differential MAL2 gene expression was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than endpoint readout. The ratios of MAL2 cDNAs from temporal cortices of AD patients and of healthy age-matched control individuals, from the frontal cortices of AD patients and of healthy age-matched control individuals, from the hippocampi of AD patients and of age-matched control individuals, and the ratios of MAL2 cDNAs from the temporal cortex and frontal cortex of AD patients and of healthy age-matched control individuals, and the ratios of MAL2 cDNAs from the hippocampus and from frontal cortex of AD patients and of healthy age-matched control individuals, respectively, were determined (relative quantification). The mRNA expression profiling between frontal cortex tissue (F) and inferior temporal cortex tissue (T) of MAL2 has been analyzed in four up to nine tissues per Braak stage. Because of the lack of high quality tissues from one donor with Braak 3 pathology, tissues of one additional donor with Braak 2 pathology were included, and because of the lack of high quality tissues from one donor with Braak 6 pathology, tissue samples of one additional donor with Braak 5 pathology were included.

For the analysis of the profiling, two general approaches have been applied. Both comparative profiling studies, frontal cortex against inferior temporal cortex as well as control against AD patients, which contribute to the complex view of the relevance of MAL2 in AD physiology, are shown in detail below.

1) Relative comparison of the mRNA expression between frontal cortex tissue and inferior temporal cortex tissue of controls and of AD patients.

This approach allowed to verify that MAL2 is either involved in the protection of the less vulnerable tissue (frontal cortex) against degeneration, or is involved in or enhances the process of degeneration in the more vulnerable tissue (inferior temporal cortex). First, a standard curve was generated to determine the efficiency of the PCR with specific primers for the gene coding for MAL2:

SEQ ID NO:5: 5'-ACCTGTAGAGATCCTCGTCATGG-3' (nucleotides 1930-1952 of SEQ ID NO:2) and
SEQ ID NO:6: 5'-TGGCCTCACTCTTACTTGTCCTT-3' (complementary nucleotides 2000-1978 of SEQ ID NO:2).

PCR amplification (95°C and 1 sec, 56°C and 5 sec, and 72°C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed a single peak at approximately 81.5°C with no visible primer dimers. Quality and size of the PCR product were determined with the DNA LabChip system (Agilent 2100 Bioanalyzer, Agilent Technologies). A single peak at the expected size of 71 bp for the gene coding for MAL2 protein was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers SEQ ID NO:7: 5'-ACTGAAGCACTACGGGCCTG-3' and SEQ ID NO:8: 5'-AGCCGTTGGTGTCTTTGCC-3' except for $MgCl_2$ (an additional 1 mM was added instead of 3 mM). Melting curve analysis revealed a single peak at approximately 87°C with no visible primer dimers. Agarose gel analysis of the PCR product showed one single band of the expected size (62 bp). (2) Ribosomal protein S9 (RPS9), using the specific primers SEQ ID NO:9: 5'-GGTCAAATTTACCCTGGCCA-3' and SEQ ID NO:10: 5'-TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers SEQ ID NO: 11: 5'-TGGAACGGTGAAGGTGACA-3' and SEQ ID NO:12: 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers SEQ ID NO:13: 5'-CGTCATGGGTGTGAACCATG-3' and SEQ ID NO:14: 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers SEQ ID NO:15: 5'-GTCGCTGGTCAGTTCGTGATT-3' and SEQ ID NO:16: 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for the gene coding for MAL2 protein and the five reference standard genes. The slopes and the intercepts of the standard curves (i.e. linear regressions) were calculated for all genes. In a second step, cDNAs from frontal cortex, temporal cortex and hippocampus, and cDNAs from frontal cortices of AD patients and of healthy control individuals, and from temporal cortices of AD patients and of healthy control individuals, respectively, were analyzed in parallel and normalized to cyclophilin B. The $C_t$ values were measured and converted to ng total brain cDNA using the corresponding standard curves:

$$10 \wedge ((C_t \text{ value - intercept}) / \text{slope}) \quad [\text{ng total brain cDNA}]$$

The values for temporal and frontal cortex and the values for hippocampus and frontal cortex MAL2 cDNAs, and the values from the frontal cortex MAL2 cDNAs of AD patients (P) and control individuals (C), and the values for temporal cortex MAL2 cDNAs of AD patients (P) and of healthy control individuals (C), respectively, were normalized to cyclophilin B and the ratios were calculated according to formulas:

$$\text{Ratio} = \frac{\text{MAL2 temporal [ng] / cyclophilin B temporal [ng]}}{\text{MAL2 frontal [ng] / cyclophilin B frontal [ng]}}$$

$$\text{Ratio} = \frac{\text{MAL2 hippocampus [ng] / cyclophilin B hippocampus [ng]}}{\text{MAL2 frontal [ng] / cyclophilin B frontal [ng]}}$$

$$\text{Ratio} = \frac{\text{MAL2 (P) temporal [ng] / cyclophilin B (P) temporal [ng]}}{\text{MAL2 (C) temporal [ng] / cyclophilin B (C) temporal [ng]}}$$

$$\text{Ratio} = \frac{\text{MAL2 (P) frontal [ng] / cyclophilin B (P) frontal [ng]}}{\text{MAL2 (C) frontal [ng] / cyclophilin B (C) frontal [ng]}}$$

In a third step, the set of reference standard genes was analyzed in parallel to determine the mean average value of the AD patient to control person temporal cortex ratios, of the AD patient to control person frontal cortex ratios, and of the temporal to frontal cortex ratios, and of the hippocampal to frontal cortex ratios of AD patients and of control persons, respectively, of expression levels of the reference standard genes for each individual brain sample. As cyclophilin B was analyzed in step 2 and step 3, and the ratio from one gene to another gene remained constant in different runs, it was possible to normalize the values for the gene coding for MAL2 protein to the mean average value of the set of reference standard genes instead of normalizing to one single gene alone. The calculation was performed by dividing the respective ratios shown above by the deviation of cyclophilin B from the mean value of all housekeeping genes. The results of such quantitative RT-PCR analysis for the gene coding for MAL2 protein are shown in Figures 2, 3, 4 and 10 and 11.

2) Comparison of the mRNA expression between controls and AD patients.

For this analysis it was proven that absolute values of real-time quantitative PCR (Lightcycler method) between different experiments at different time points are consistent enough to be used for quantitive comparisons without usage of calibrators. Cyclophilin was used as a standard for normalization in any of the qPCR experiments for more than 100 tissues. Between others it was found to be the most consistently expressed housekeeping gene in our normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-conficence level was applied. This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations. A detailed analysis of absolute values for MAL2 was performed. Therefore, absolute levels of MAL2 were used after relative normalization with cyclophilin. The median as well as the 98 %-confidence level was calculated for the control group (Braak 0 - Braak 3) and the patient group (Braak 4 - Braak 6), respectively. The same analysis was done redefining the control group (Braak 0 - Braak 2) and the patient group (Braak 3 - Braak 6) as well as redefining the control group (Braak 0 - Braak 1) and the patient group (Braak 2 - Braak 6). The latter analysis was aimed to identify early onset of mRNA expression differences between controls and AD patients. In another view of this analysis, three groups comprising Braak stages 0-1, Braak stages 2-3, and Braak stages 4-6, respectively, were compared to each other in order to identify tendencies of gene expression regulation as well as early onset differences. Said analysis as described above is shown in Figure 12.

(v) Immunoblotting:

Total protein extract was obtained from H4APPsw cells expressing MAL2-myc by homogenization in 1 ml RIPA buffer (150 mM sodium chloride, 50 mM tris-HCl, pH7.4, 1 mM ethylenediamine-tetraacetic acid, 1 mM phenylmethylsulfonyl flouride, 1% Triton X-100, 1% sodium deoxycholic acid, 1% sodium dodecylsulfate, 5 μg/ml of aprotinin, 5 μg/ml of leupeptin) on ice. After centrifuging twice for 5 min at 3000 rpm at 4°C, the supernatant was diluted five-fold in SDS-loading buffer. Aliquots of 12 μl of the diluted sample were resolved by SDS-PAGE (8% polyacrylamide) and transferred to PVDF Western Blotting membranes (Boehringer Mannheim). The blots were probed with rabbit polyclonal anti- myc antibodies (MBL, 1:1000) followed by horseradish peroxidase-coupled goat anti-rabbit IgG antiserum (Santa Cruz sc-2030, diluted 1:5000) and developed with the ECL chemoluminescence detection kit (Amersham Pharmacia) (Figure 13).

(vi) Immunofluorescence Analysis (IF):

For the immunofluorescence staining of MAL2 protein in cells, a human neuroglioma cell line was used (H4 cells) which stably expresses the human APP695 isoform carrying the Swedish mutation (K670N, M671L) (H4APPsw cells).
The H4APPsw cells were transduced with a pFB-Neo vector (Stratagene, #217561) containing the coding sequence of MAL2 (MAL2 cds) (SEQ ID NO:4, 531 bp) and a myc-tag (pFB-Neo-MAL2cds-myc, MAL2-myc vector, 7135 bp, EcoRI/XhoI) under the control of a strong CMV promotor. For the generation of the MAL2-myc vector, the MAL2cds-myc sequence was introduced into the EcoRI/XhoI restriction sites of the multiple cloning site (MCS) of the pFB-Neo vector. For transduction of the H4APPsw cells with the MAL2-myc vector the retroviral expression system ViraPort from Stratagene was used.
The myc-tagged MAL2 over-expressing cells (H4APPsw-MAL2cds-myc) were seeded onto glass cover slips in a 24 well plate (Nunc, Roskilde, Denmark; #143982) at a density of $5x10^4$ cells and incubated at 37°C at 5% $CO_2$ over night. To fix the cells onto the cover slip, medium was removed and chilled methanol (-20°C) was added. After an incubation period of 15 minutes at -20°C, methanol was removed and the fixed cells were blocked for 1 hour in blocking solution (200µl PBS/ 5% BSA/ 3% goat serum) at room temperature. The first antibody (polyclonal anti-myc antibody, rabbit, 1:5000, MBL) and DAPI (DNA-stain, 0.05µg/ml, 1:1000) in PBS / 1% goat serum was added and incubated for 1 hour at room temperature. After removing the first antibody, the fixed cells were washed 3 times with PBS for 5 minutes. The second antibody (Cy3-conjugated anti-rabbit antibody, 1:1000, Amersham Pharmacia, Germany) was applied in blocking solution and incubated for 1 hour at room temperature. The cells were washed 3 times in PBS for 5 minutes. Coverslips were mounted onto microscope slides using Permafluor (Beckman Coulter) and stored over night at 4°C to harden the mounting media. Cells were visualized using microscopic dark field epifluorescence and bright field phase contrast illumination conditions (IX81, Olympus Optical). Microscopic images (Figure 14) were digitally captured with a PCO SensiCam and analysed using the appropriate software (AnalySiS, Olympus Optical).

(vii) Immunohistochemistry:

For immunofluorescence staining of MAL2 in human brain, frozen sections were prepared with a cryostat (Leica CM3050S) from post-mortem frontal cortex of a donor person and fixed in 4% PFA 20 min at room temperature. After washing in PBS, the sections were pre-incubated with blocking buffer (10% normal goat serum, 0.2% Triton X-100 in PBS) for 30 min and then incubated with affinity-purified rabbit polyclonal anti-MAL2 antisera (1:60 diluted in blocking buffer; Davids Biotechnology, Regensburg) and with a mouse monoclonal anti-CNPase antibody (C5P22; Sigma) overnight at 4°C. After rinsing three times in 0.1% Triton X-100/PBS, the sections were incubated with FITC-conjugated goat anti-rabbit IgG antisera (1:150 diluted in 1% BSA/PBS) and with a Cy3-conjugated goat anti-mouse IgG antiserum (1:600) for 2 hours at room temperature and then again washed in PBS. Staining of the nuclei was performed by incubation of the sections with 5µM DAPI in PBS for 3 min (blue signal). In order to block the autofluoresence of lipofuscin in human brain, the sections were treated with 1% Sudan Black B in 70% ethanol for 2-10 min at room temperature and then sequentially dipped in 70% ethanol, destilled water and PBS. The sections were coverslipped with 'Vectrashield' mounting medium (Vector Laboratories, Burlingame, CA) and observed under an inverted microscope (IX81, Olympus Optical). The digital images were captured with the appropriate software (AnalySiS, Olympus Optical) (Figure 15).

## Claims

1. A method of diagnosing or prognosticating Alzheimer's disease in a subject, or determining whether a subject is at increased risk of developing said disease, comprising determining a level and/or an activity of

   (i) a transcription product of the gene coding for MAL2 protein, and/or
   (ii) a translation product of the gene coding for MAL2 protein,

   in a sample obtained from said subject and comparing said level and/or said activity of said transcription product and/or said translation product to a reference value representing a known disease status and/or to a reference value representing a known health status, and said level and/or said activity is varied compared to a reference value representing a known health status, and/or is similar or equal to a reference value representing a known disease status, thereby diagnosing or prognosticating Alzheimer's disease in said subject, or determining whether said subject is at increased risk of developing said disease.

2. Use of the recombinant, non-human animal comprising a non-native gene sequence coding for MAL2 for screening, testing, and validating compounds, agents, and modulators in the development of diagnostics and therapeutics to treat neurodegenerative diseases, in particular Alzheimer's disease.

3. An in vitro assay for screening for a modulator of neurodegenerative diseases, in particular Alzheimer's disease, or related diseases or disorders of one or more substances selected from the group consisting of

(i) a gene coding for MAL2 protein, and/or
(ii) a transcription product of the gene coding for MAL2 protein, and/or
(iii) a translation product of the gene coding for MAL2 protein,

said method comprising:

(a) contacting a cell with a test compound;
(b) measuring the activity and/or level of one or more substances recited in (i) to (iii);
(c) measuring the activity and/or level of one or more substances recited in (i) to (iii) in a control cell not contacted with said test compound; and

comparing the levels and/or activities of the substance in the cells of step (b) and (c), wherein an alteration in the activity and/or level of substances in the contacted cells indicates that the test compound is a modulator of said diseases or disorders.

4. A method of screening for a modulator of neurodegenerative diseases, in particular Alzheimer's disease, or related diseases or disorders of one or more substances selected from the group consisting of

(i) the gene coding for MAL2 protein, and/or
(ii) a transcription product of the gene coding for MAL2 protein, and/or
(iii) a translation product of the gene coding for MAL2 protein,

said method comprising:

(a) administering a test compound to a test animal which is predisposed to developing or has already developed symptoms of a neurodegenerative disease or related diseases or disorders in respect of the substances recited in (i) to (iii);
(b) measuring the activity and/or level of one or more substances recited in (i) to (iii);
(c) measuring the activity and/or level of one or more substances recited in (i) or (iii) in a matched control animal which is predisposed to developing or has already developed symptoms of a neurodegenerative disease or related diseases or disorders in respect to the substances recited in (i) to (iii) and to which animal no such test compound has been administered;
(d) comparing the activity and/or level of the substance in the animals of step (b) and (c), wherein an alteration in the activity and/or level of substances in the test animal indicates that the test compound is a modulator of said diseases or disorders.

5. The method according to claim 4 wherein said test animal and/or said control animal is a recombinant animal which expresses MAL2under the control of a transcriptional control element which is not the native MAL2 gene transcriptional control element.

6. Use of a protein molecule of SEQ ID NO: 1, said protein molecule being a translation product of the gene coding for MAL2 as a diagnostic target for detecting Alzheimer's disease.

7. Use of a protein molecule of SEQ ID NO: 1, said protein molecule being a translation product of the gene coding for MAL2 as a screening target for reagents or compounds preventing, or treating, or ameliorating Alzheimer's disease.

8. Use of an antibody specifically immunoreactive with an immunogen, wherein said immunogen is a translation product of a gene coding for MAL2, SEQ ID NO: 1 for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to Alzheimer's disease.

## Patentansprüche

1. Verfahren zum Diagnostizieren oder Prognostizieren der Alzheimer-Krankheit bei einem Patienten oder zum Bestimmen, ob ein Patient ein erhöhtes Risiko hat, diese Krankheit zu entwickeln, umfassend das Bestimmen einer Konzentration und/oder einer Aktivität von:

   (i) einem Transcriptionsprodukt des Gens, das für das MAL2-Protein codiert; und/oder
   (ii) einem Translationsprodukt des Gens, das für das MAL2-Protein codiert;

   in einer Probe, die von dem Patienten erhalten wurde, und Vergleichen der Konzentration und/oder der Aktivität des Transcriptionsprodukts und/oder des Translationsprodukts mit einem Referenzwert, der einen bekannten Krankheitszustand darstellt, und/oder mit einem Referenzwert, der einen bekannten Gesundheitszustand darstellt, wobei die Konzentration und/oder die Aktivität von einem Referenzwert, der einen bekannten Gesundheitszustand darstellt, abweicht und/oder ähnlich oder gleich ist wie ein Referenzwert, der einen bekannten Krankheitszustand darstellt, wodurch die Alzheimer-Krankheit bei dem Patienten diagnostiziert oder prognostiziert wird oder bestimmt wird, ob der Patient ein erhöhtes Risiko hat, diese Krankheit zu entwickeln.

2. Verwendung des rekombinanten nichthumanen Tiers, das eine nichtnative Gensequenz umfasst, die für MAL2 codiert, zum Durchmustern, Testen und Bewerten von Verbindungen, Mitteln und Modulatoren bei der Entwicklung von Diagnostika und Therapeutika zur Behandlung von neurodegenerativen Krankheiten, insbesondere Alzheimer-Krankheit.

3. In-vitro-Assay zum Suchen nach einem Modulator von neurodegenerativen Krankheiten, insbesondere Alzheimer-Krankheit, oder verwandten Krankheiten oder Störungen durch Screening von einer oder mehreren Substanzen, die aus der Gruppe ausgewählt sind, die aus Folgenden besteht:

   (i) einem Gen, das für MAL2-Protein codiert; und/oder
   (ii) einem Transcriptionsprodukt des Gens, das für MAL2-Protein codiert; und/oder
   (iii) einem Translationsprodukt des Gens, das für MAL2-Protein codiert;

   wobei das Verfahren Folgendes umfasst:

   (a) In-Kontakt-Bringen einer Zelle mit einer Testverbindung;
   (b) Messen der Aktivität und/oder der Konzentration von einer oder mehreren Substanzen, die in (i) bis (iii) genannt sind;
   c) Messen der Aktivität und/oder der Konzentration von einer oder mehreren Substanzen, die in (i) bis (iii) genannt sind, in einer Kontrollzelle, die nicht mit der Testverbindung in Kontakt gebracht wurde; und

   Vergleichen der Konzentrationen und/oder Aktivitäten der Substanz in den Zellen der Schritte (b) und (c), wobei eine Änderung der Aktivität und/oder der Konzentration der Substanzen in den kontaktierten Zellen anzeigt, dass die Testverbindung ein Modulator der Krankheiten oder Störungen ist.

4. Verfahren zum Suchen nach einem Modulator von neurodegenerativen Krankheiten, insbesondere Alzheimer-Krankheit, oder verwandten Krankheiten oder Störungen durch Screening von einer oder mehreren Substanzen, die aus der Gruppe ausgewählt sind, die aus Folgenden besteht:

   (i) einem Gen, das für MAL2-Protein codiert; und/oder
   (ii) einem Transcriptionsprodukt des Gens, das für MAL2-Protein codiert; und/oder
   (iii) einem Translationsprodukt des Gens, das für MAL2-Protein codiert;

   wobei das Verfahren Folgendes umfasst:

   (a) Verabreichen einer Testverbindung an ein Versuchstier, das prädisponiert ist, Symptome einer neurodegenerativen Krankheit oder von verwandten Krankheiten oder Störungen zu entwickeln, oder diese bereits entwickelt hat, in Bezug auf die in (i) bis (iii) genannten Substanzen;
(b) Messen der Aktivität und/oder der Konzentration von einer oder mehreren Substanzen, die in (i) bis (iii) genannt sind;
(c) Messen der Aktivität und/oder der Konzentration von einer oder mehreren Substanzen, die in (i) bis (iii) genannt sind, bei einem darauf abgestimmten Kontrolltier, das prädisponiert ist, Symptome einer neurodegenerativen Krankheit oder von verwandten Krankheiten oder Störungen zu entwickeln, oder diese bereits entwickelt hat, in Bezug auf die in (i) bis (iii) genannten Substanzen, wobei diesem Tier keine solche Testverbindung verabreicht wurde;
(d) Vergleichen der Konzentration und/oder Aktivität der Substanz in den Tieren der Schritte (b) und (c), wobei eine Änderung der Aktivität und/oder der Konzentration der Substanzen in dem Versuchstier anzeigt, dass die Testverbindung ein Modulator der Krankheiten oder Störungen ist.

**5.** Verfahren gemäß Anspruch 4, wobei das Versuchstier und/oder das Kontrolltier ein rekombinantes Tier ist, das MAL2 unter der Kontrolle eines Transcriptionskontrollelements, bei dem es sich nicht um das native Transcriptionskontrollelement des MAL2-Gens handelt, exprimiert.

**6.** Verwendung eines Proteinmoleküls von SEQ ID Nr. 1, wobei das Proteinmolekül ein Translationsprodukt des Gens ist, das für MAL2 codiert, als diagnostisches Target zum Nachweisen der Alzheimer-Krankheit.

**7.** Verwendung eines Proteinmoleküls von SEQ ID Nr. 1, wobei das Proteinmolekül ein Translationsprodukt des Gens ist, das für MAL2 codiert, als Screening-Target für Reagentien oder Verbindungen, die die Alzheimer-Krankheit verhindern oder behandeln oder lindern.

**8.** Verwendung eines Antikörpers, der gegenüber einem Immunogen spezifisch immunreaktiv ist, wobei das Immunogen ein Translationsprodukt eines für MAL2 codierenden Gens, SEQ ID Nr. 1, ist zum Nachweis des pathologischen Zustands einer Zelle in einer Probe, die von einem Patienten erhalten wurde, umfassend das immunocytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt, der mit der Alzheimer-Krankheit in Zusammenhang steht.

## Revendications

**1.** Procédé permettant de diagnostiquer ou de pronostiquer la maladie d'Alzheimer chez un sujet, ou de déterminer si un sujet présente un haut risque de développer ladite maladie, comprenant la détermination d'un niveau et/ou d'une activité :

(i) d'un produit de transcription du gène codant la protéine MAL2, et/ou
(ii) d'un produit de traduction du gène codant la protéine MAL2,
dans un échantillon obtenu à partir dudit sujet, et la comparaison dudit niveau et/ou de ladite activité dudit produit de transcription et/ou dudit produit de traduction à une valeur de référence représentant un état pathologique connu et/ou à une valeur de référence représentant un état de santé connu, ledit niveau et/ou ladite activité étant différent d'une valeur de référence représentant un état de santé connu et/ou étant similaire ou égal à une valeur de référence représentant un état pathologique connu, pour ainsi diagnostiquer ou pronostiquer la maladie d'Alzheimer chez ledit sujet, ou déterminer si ledit sujet présente un haut risque de développer ladite maladie.

**2.** Utilisation de l'animal non humain transgénique comprenant une séquence génique non native codant la protéine MAL2, pour le criblage, le test et la validation de composés, d'agents et de modulateurs dans la mise au point de techniques diagnostiques et thérapeutiques permettant de traiter les maladies neurodégénératives, en particulier la maladie d'Alzheimer.

**3.** Dosage in vitro permettant de cribler un modulateur de maladies neurodégénératives, en particulier de la maladie d'Alzheimer, ou de troubles ou maladies apparentés d'une ou plusieurs substances choisies dans le groupe formé par :

(i) un gène codant la protéine MAL2, et/ou

(ii) un produit de transcription du gène codant la protéine MAL2, et/ou
(iii)un produit de traduction du gène codant la protéine MAL2,

ledit procédé comprenant les étapes consistant à :

(a) mettre en contact une cellule avec un composé d'essai ;
(b) mesurer l'activité et/ou le niveau d'une ou plusieurs substances citées aux points (i) à (iii) ;
(c) mesurer l'activité et/ou le niveau d'une ou plusieurs substances citées aux points (i) à (iii) dans une cellule témoin n'ayant pas été mise en contact avec ledit composé d'essai ; et

comparer le niveau et/ou l'activité des substances mesurées dans les cellules aux étapes (b) et (c), dans lequel une variation de l'activité et/ou du niveau des substances dans les cellules mises en contact indique que le composé d'essai est un modulateur desdits troubles ou maladies.

**4.** Procédé de criblage d'un modulateur de maladies neurodégénératives, en particulier de la maladie d'Alzheimer, ou de troubles ou maladies apparentés d'une ou plusieurs substances choisies dans le groupe formé par :

(i) le gène codant la protéine MAL2, et/ou
(ii) un produit de transcription du gène codant la protéine MAL2, et/ou
(iii)un produit de traduction du gène codant la protéine MAL2,

ledit procédé comprenant les étapes consistant à :

(a) administrer un composé d'essai à un animal d'essai qui est prédisposé à développer ou qui a déjà développé des symptômes d'une maladie neurodégénérative ou de troubles ou maladies apparentés au niveau des substances citées aux points (i) à (iii) ;
(b) mesurer l'activité et/ou le niveau d'une ou plusieurs substances citées aux points (i) à (iii) ;
(c) mesurer l'activité et/ou le niveau d'une ou plusieurs substances citées aux points (i) à (iii) chez un animal témoin correspondant qui est prédisposé à développer ou qui a déjà développé des symptômes d'une maladie neurodégénérative ou de troubles ou maladies apparentés au niveau des substances citées aux points (i) à (iii) et auquel aucun de ces composés d'essai n'a été administré ;
(d) comparer l'activité et/ou le niveau des substances mesurés chez les animaux aux étapes (b) et (c), dans lequel une variation de l'activité et/ou du niveau des substances chez l'animal d'essai indique que le composé d'essai est un modulateur desdits troubles ou maladies.

**5.** Procédé selon la revendication 4, dans lequel ledit animal d'essai et/ou ledit animal témoin est un animal transgénique qui exprime la protéine MAL2 sous la commande d'un élément de régulation transcriptionnel qui n'est pas l'élément de régulation transcriptionnel du gène MAL2 natif.

**6.** Utilisation d'une molécule protéique de séquence SEQ ID N° 1, ladite molécule protéique étant un produit de traduction du gène codant la protéine MAL2, en tant que cible diagnostique permettant de détecter la maladie d'Alzheimer.

**7.** Utilisation d'une molécule protéique de séquence SEQ ID N° 1, ladite molécule protéique étant un produit de traduction du gène codant la protéine MAL2, en tant que cible de criblage pour des réactifs ou des composés prévenant, traitant ou améliorant la maladie d'Alzheimer.

**8.** Utilisation d'un anticorps spécifiquement immunoréactif envers un agent immunogène, dans laquelle ledit agent immunogène est un produit de traduction d'un gène codant la protéine MAL2, SEQ ID N° 1, pour détecter l'état pathologique d'une cellule dans un échantillon obtenu à partir d'un sujet, consistant à colorer immunocytochimiquement ladite cellule avec ledit anticorps, dans laquelle une variation du degré de coloration ou du profil de coloration dans ladite cellule par rapport à une cellule représentant un état de santé connu indique un état pathologique de ladite cellule qui a trait à la maladie d'Alzheimer.

Fig. 1: Identification of differentially expressed genes in a fluorescence differential display screen

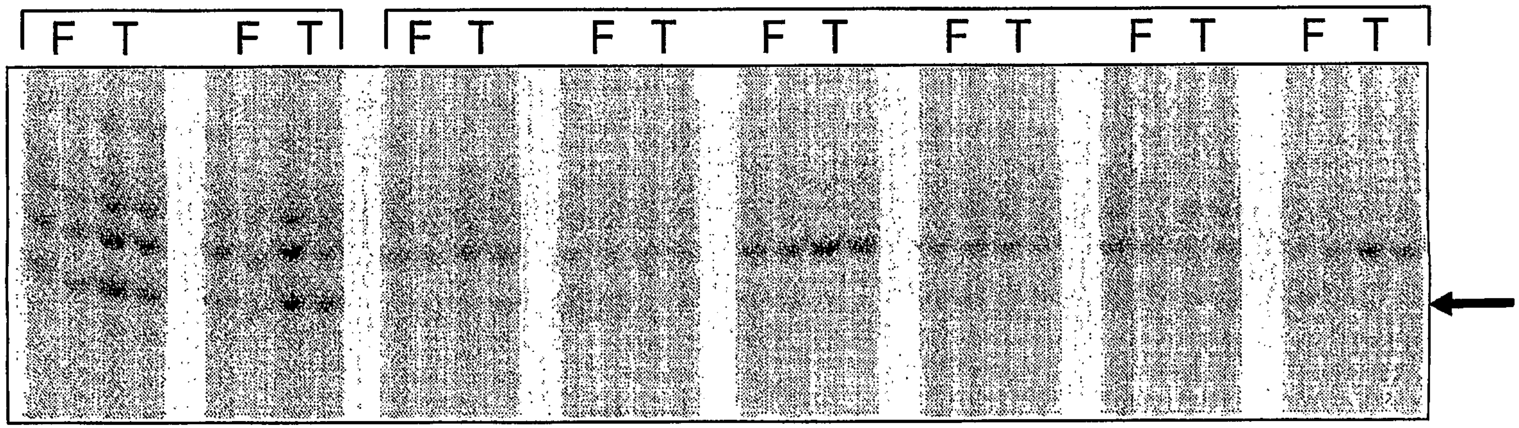

Fig. 2: Verification of differential expression of human MAL2 by quantitative RT-PCR

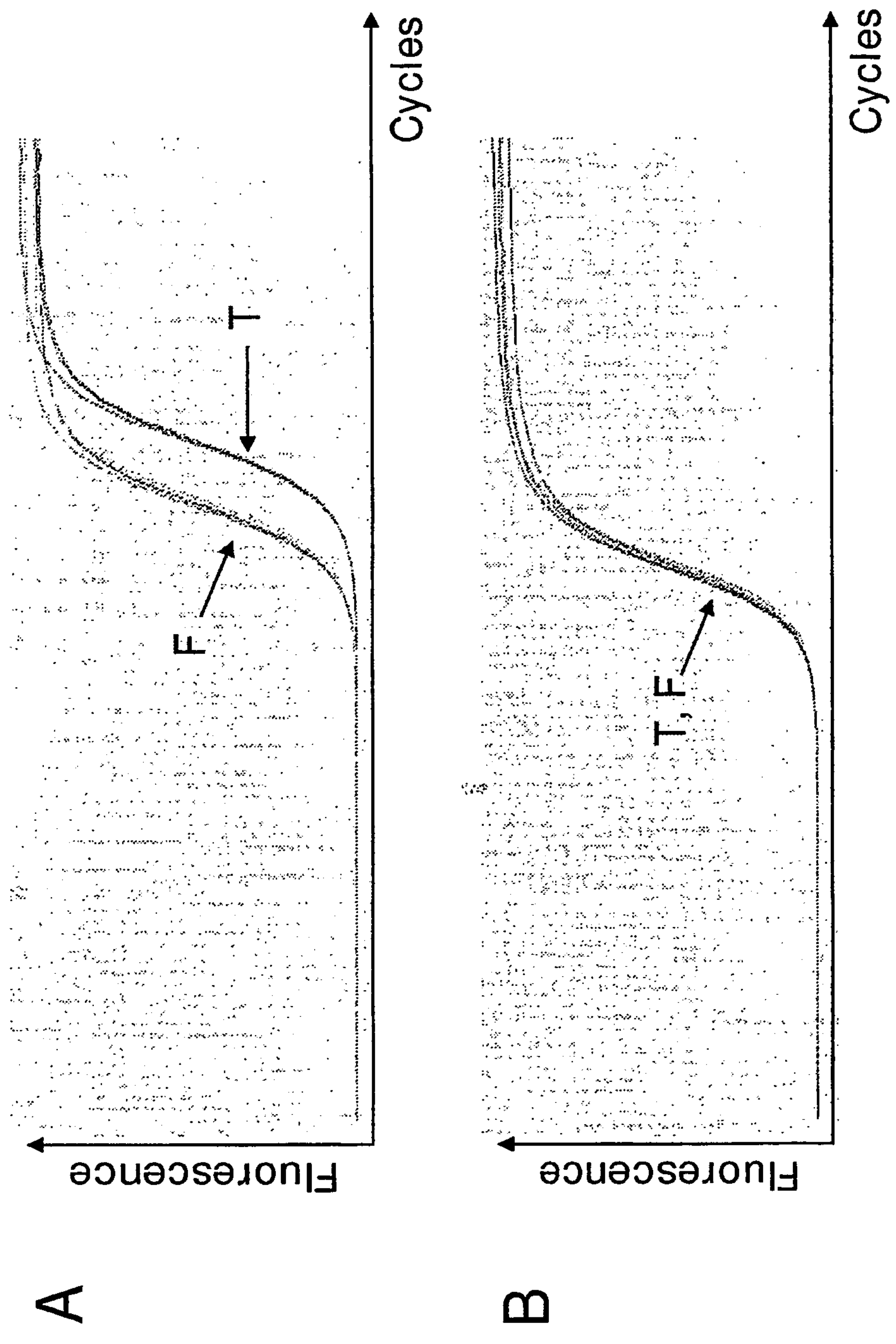

Fig. 3: Verification of differential expression of human MAL2 by quantitative RT-PCR

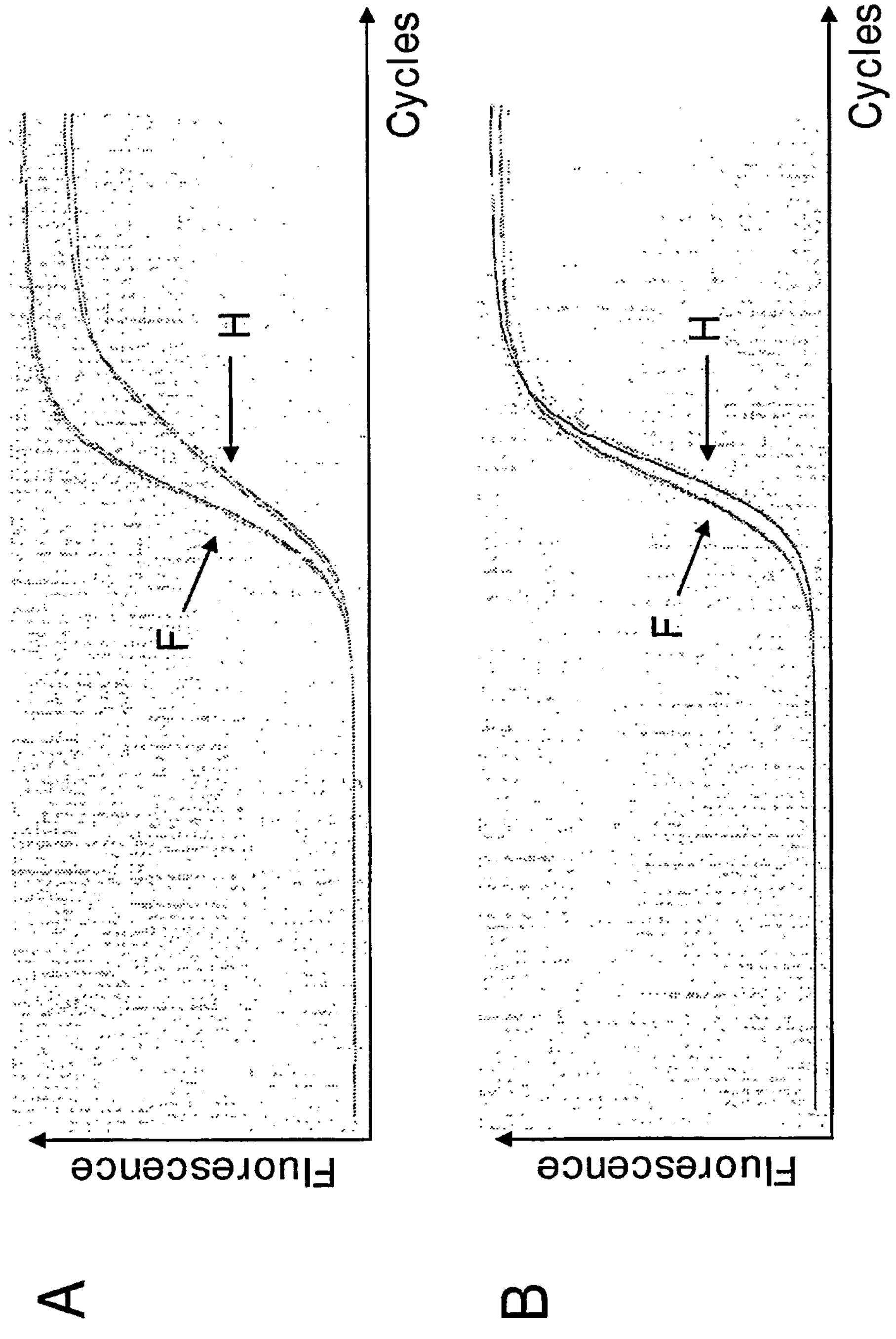

Fig. 4: Verification of differential expression of MAL2 by quantitative PCR

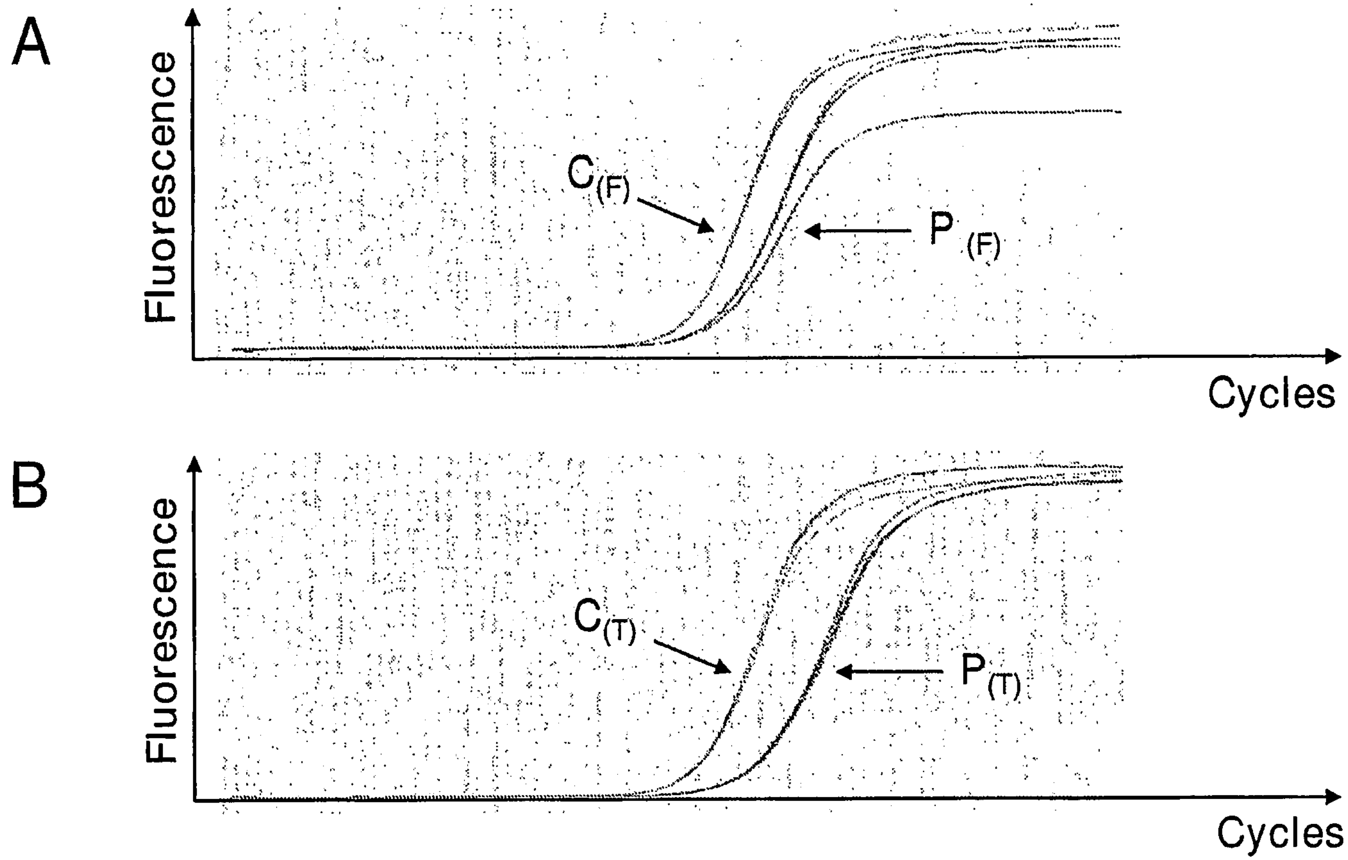

Fig. 5 : SEQ ID NO: 1,
amino acid sequence of
human MAL2 protein

Length: 176 aa

```
  1 MSAGGASVPP PPNPAVSFPP PRVTLPAGPD ILRTYSGAFV CLEILFGGLV
 51 WILVASSNVP LPLLQGWVMF VSVTAFFFSL LFLGMFLSGM VAQIDANWNF
101 LDFAYHFTVF VFYFGAFLLE AAATSLHDLH CNTTITGQPL LSDNQYNINV
151 AASIFAFMTT ACYGCSLGLA LRRWRP
```

Fig. 6: SEQ ID NO: 2,
nucleotide sequence of
human MAL2 cDNA

Length: 2808 bp

```
   1 GGCGGCGGCG GCAGGAGCCC GGGAGGCGGA GGCGGGAGGC GGCGGCGGCG
  51 CGCGGAGACG CAGCAGCGGC AGCGGCAGCA TGTCGGCCGG CGGAGCGTCA
 101 GTCCCGCCGC CCCCGAACCC CGCCGTGTCC TTCCCGCCGC CCCGGGTCAC
 151 CCTGCCCGCC GGCCCCGACA TCCTGCGGAC CTACTCGGGC GCCTTCGTCT
 201 GCCTGGAGAT TCTGTTCGGG GGTCTTGTCT GGATTTTGGT TGCCTCCTCC
 251 AATGTTCCTC TACCTCTACT ACAAGGATGG GTCATGTTTG TGTCCGTGAC
 301 AGCGTTTTTC TTTTCGCTCC TCTTTCTGGG CATGTTCCTC TCTGGCATGG
 351 TGGCTCAAAT TGATGCTAAC TGGAACTTCC TGGATTTTGC CTACCATTTT
 401 ACAGTATTTG TCTTCTATTT TGGAGCCTTT TTATTGGAAG CAGCAGCCAC
 451 ATCCCTGCAT GATTTGCATT GCAATACAAC CATAACCGGG CAGCCACTCC
 501 TGAGTGATAA CCAGTATAAC ATAAACGTAG CAGCCTCAAT TTTTGCCTTT
 551 ATGACGACAG CTTGTTATGG TTGCAGTTTG GGTCTGGCTT TACGAAGATG
 601 GCGACCGTAA CACTCCTTAG AAACTGGCAG TCGTATGTTA GTTTCACTTG
 651 TCTACTTTAT ATGTCTGATC AATTTGGATA CCATTTTGTC CAGATGCAAA
 701 AACATTCCAA AAGTAATGTG TTTAGTAGAG AGAGACTCTA AGCTCAAGTT
 751 CTGGTTTATT TCATGGATGG AATGTTAATT TTATTATGAT ATTAAAGAAA
 801 TGGCCTTTTA TTTTACATCT CTCCCCTTTT TCCCTTTCCC CCTTTATTTT
 851 CCTCCTTTTC TTTCTGAAAG TTTCCTTTTA TGTCCATAAA ATACAAATAT
 901 ATTGTTCATA AAAAATTAGT ATCCCTTTTG TTTGGTTGCT GAGTCACCTG
 951 AACCTTAATT TTAATTGGTA ATTACAGCCC CTAAAAAAAA CACATTTCAA
1001 ATAGGCTTCC CACTAAACTC TATATTTTAG TGTAAACCAG GAATTGGCAC
1051 ACTTTTTTTA GAATGGGCCA GATGGTAAAT ATTTATGCTT CACGGTCCAT
1101 ACAGTCTCTG TCACAACTAT TCAGTTCTGC TAGTATAGCG TGAAAGCAGC
1151 TATACACAAT ACAGAAATGA ATGAGTGTGG TTATGTTCTA ATAAAACTTA
1201 TTTATAAAAA CAAGGGGAGG CTGGGTTTAG CCTGTGGGCC ATAGTTTGTC
1251 AACCACTGGT GTAAAACCTT AGTTATATAT GATCTGCATT TTCTTGAACT
1301 GATCATTGAA AACTTATAAA CCTAACAGAA AAGCCACATA ATATTTAGTG
1351 TCATTATGCA ATAATCACAT TGCCTTTGTG TTAATAGTCA AATACTTACC
1401 TTTGGAGAAT ACTTACCTTT GGAGGAATGT ATAAAATTTC TCAGGCAGAG
1451 TCCTGGATAT AGGAAAAAGT AATTTATGAA GTAAACTTCA GTTGCTTAAT
1501 CAAACTAATG ATAGTCTAAC AACTGAGCAA GATCCTCATC TGAGAGTGCT
1551 TAAAATGGGA TCCCCAGAGA CCATTAACCA ATACTGGAAC TGGTATCTAG
1601 CTACTGATGT CTTACTTTGA GTTTATTTAT GCTTCAGAAT ACAGTTGTTT
1651 GCCCTGTGCA TGAATATACC CATATTTGTG TGTGGATATG TGAAGCTTTT
1701 CCAAATAGAG CTCTCAGAAG AATTAAGTTT TTACTTCTAA TTATTTTGCA
1751 TTACTTTGAG TTAAATTTGA ATAGAGTATT AAATATAAAG TTGTAGATTC
1801 TTATGTGTTT TTGTATTAGC CCAGACATCT GTAATGTTTT TGCACTGGTG
1851 ACAGACAAAA TCTGTTTTAA AATCATATCC AGCACAAAAA CTATTTCTGG
1901 CTGAATAGCA CAGAAAAGTA TTTTAACCTA CCTGTAGAGA TCCTCGTCAT
1951 GGAAAGGTGC CAAACTGTTT TGAATGGAAG GACAAGTAAG AGTGAGGCCA
2001 CAGTTCCCAC CACACGAGGG CTTTTGTATT GTTCTACTTT TTCAGCCCTT
2051 TACTTTCTGG CTGAAGCATC CCCTTGGAGT GCCATGTATA AGTTGGGCTA
2101 TTAGAGTTCA TGGAACATAG AACAACCATG AATGAGTGGC ATGATCCGTG
2151 CTTAATGATC AAGTGTTACT TATCTAATAA TCCTCTAGAA AGAACCCTGT
2201 TAGATCTTGG TTTGTGATAA AAATATAAAG ACAGAAGACA TGAGGAAAAA
```

2251 CAAAAGGTTT GAGGAAATCA GGCATATGAC TTTATACTTA ACATCAGATC
2301 TTTTCTATAA TATCCTACTA CTTTGGTTTT CCTAGCTCCA TACCACACAC
2351 CTAAACCTGT ATTATGAATT ACATATTACA AAGTCATAAA TGTGCCATAT
2401 GGATATACAG TACATTCTAG TTGGAATCGT TTACTCTGCT AGAATTTAGG
2451 TGTGAGATTT TTTGTTTCCC AGGTATAGCA GGCTTATGTT TGGTGGCATT
2501 AAATTGGTTT CTTTAAAATG CTTTGGTGGC ACTTTTGTAA ACAGATTGCT
2551 TCTAGATTGT TACAAACCAA GCCTAAGACA CATCTGTGAA TACTTAGATT
2601 TGTAGCTTAA TCACATTCTA GACTTGTGAG TTGAATGACA AAGCAGTTGA
2651 ACAAAAATTA TGGCATTTAA GAATTTAACA TGTCTTAGCT GTAAAAATGA
2701 GAAAGTGTTG GTTGGTTTTA AAATCTGGTA ACTCCATGAT GAAAAGAAAT
2751 TTATTTTATA CGTGTTATGT CTCTAATAAA GTATTCATTT GATAAAAAAA
2801 AAAAAAAA

Fig. 7: SEQ ID NO: 3

Length: 270 bp

```
  1 TGGTGGCACT TTTGTAAACA GATTGCTTCT AGATTGTTAC AAACCAAGCC
 51 TAAGACACAT CTGTGAATAC TTAGATTTGT AGCTTAATCA CATTCTAGAC
101 TTGTGAGTTG AATGACAAAG CAGTTGAACA AAAATTATGG CATTTAAGAA
151 TTTAACATGT CTTAGCTGTA AAAATGAGAA AGTGTTGGTT GGTTTTAAAA
201 TCTGGTAACT CCATGATGGA AAGAAATTTA TTTTATACGT GTTATGTCTC
251 TAATAAAGTA TTCATTTGAT
```

Fig. 8: SEQ ID NO: 4,
nucleotide sequence of
human MAL2 coding sequence

Length: 531 bp

```
  1  ATGTCGGCCG GCGGAGCGTC AGTCCCGCCG CCCCCGAACC CCGCCGTGTC
 51  CTTCCCGCCG CCCCGGGTCA CCCTGCCCGC CGGCCCCGAC ATCCTGCGGA
101  CCTACTCGGG CGCCTTCGTC TGCCTGGAGA TTCTGTTCGG GGGTCTTGTC
151  TGGATTTTGG TTGCCTCCTC CAATGTTCCT CTACCTCTAC TACAAGGATG
201  GGTCATGTTT GTGTCCGTGA CAGCGTTTTT CTTTTCGCTC CTCTTTCTGG
251  GCATGTTCCT CTCTGGCATG GTGGCTCAAA TTGATGCTAA CTGGAACTTC
301  CTGGATTTTG CCTACCATTT TACAGTATTT GTCTTCTATT TTGGAGCCTT
351  TTTATTGGAA GCAGCAGCCA CATCCCTGCA TGATTTGCAT TGCAATACAA
401  CCATAACCGG GCAGCCACTC CTGAGTGATA ACCAGTATAA CATAAACGTA
451  GCAGCCTCAA TTTTTGCCTT TATGACGACA GCTTGTTATG GTTGCAGTTT
501  GGGTCTGGCT TTACGAAGAT GGCGACCGTA A
```

# Fig. 9: Alignment of SEQ ID NO: 2 with SEQ ID NO: 3

Length: 270 bp

```
                 .         .         .         .         .
   1 TGGTGGCACTTTTGTAAACAGATTGCTTCTAGATTGTTACAAACCAAGCC 50
     ||||||||||||||||||||||||||||||||||||||||||||||||||
2524 TGGTGGCACTTTTGTAAACAGATTGCTTCTAGATTGTTACAAACCAAGCC 2573
                 .         .         .         .         .
  51 TAAGACACATCTGTGAATACTTAGATTTGTAGCTTAATCACATTCTAGAC 100
     ||||||||||||||||||||||||||||||||||||||||||||||||||
2574 TAAGACACATCTGTGAATACTTAGATTTGTAGCTTAATCACATTCTAGAC 2623
                 .         .         .         .         .
 101 TTGTGAGTTGAATGACAAAGCAGTTGAACAAAAATTATGGCATTTAAGAA 150
     ||||||||||||||||||||||||||||||||||||||||||||||||||
2624 TTGTGAGTTGAATGACAAAGCAGTTGAACAAAAATTATGGCATTTAAGAA 2673
                 .         .         .         .         .
 151 TTTAACATGTCTTAGCTGTAAAAATGAGAAAGTGTTGGTTGGTTTTAAAA 200
     ||||||||||||||||||||||||||||||||||||||||||||||||||
2674 TTTAACATGTCTTAGCTGTAAAAATGAGAAAGTGTTGGTTGGTTTTAAAA 2723
                 .         .         .         .         .
 201 TCTGGTAACTCCATGATGGAAAGAAATTTATTTTATACGTGTTATGTCTC 250
     |||||||||||||||||| |||||||||||||||||||||||||||||||
2724 TCTGGTAACTCCATGATGAAAAGAAATTTATTTTATACGTGTTATGTCTC 2773
                 .         .
 251 TAATAAAGTATTCATTTGAT 270
     ||||||||||||||||||||
2774 TAATAAAGTATTCATTTGAT 2793
```

Fig. 10:

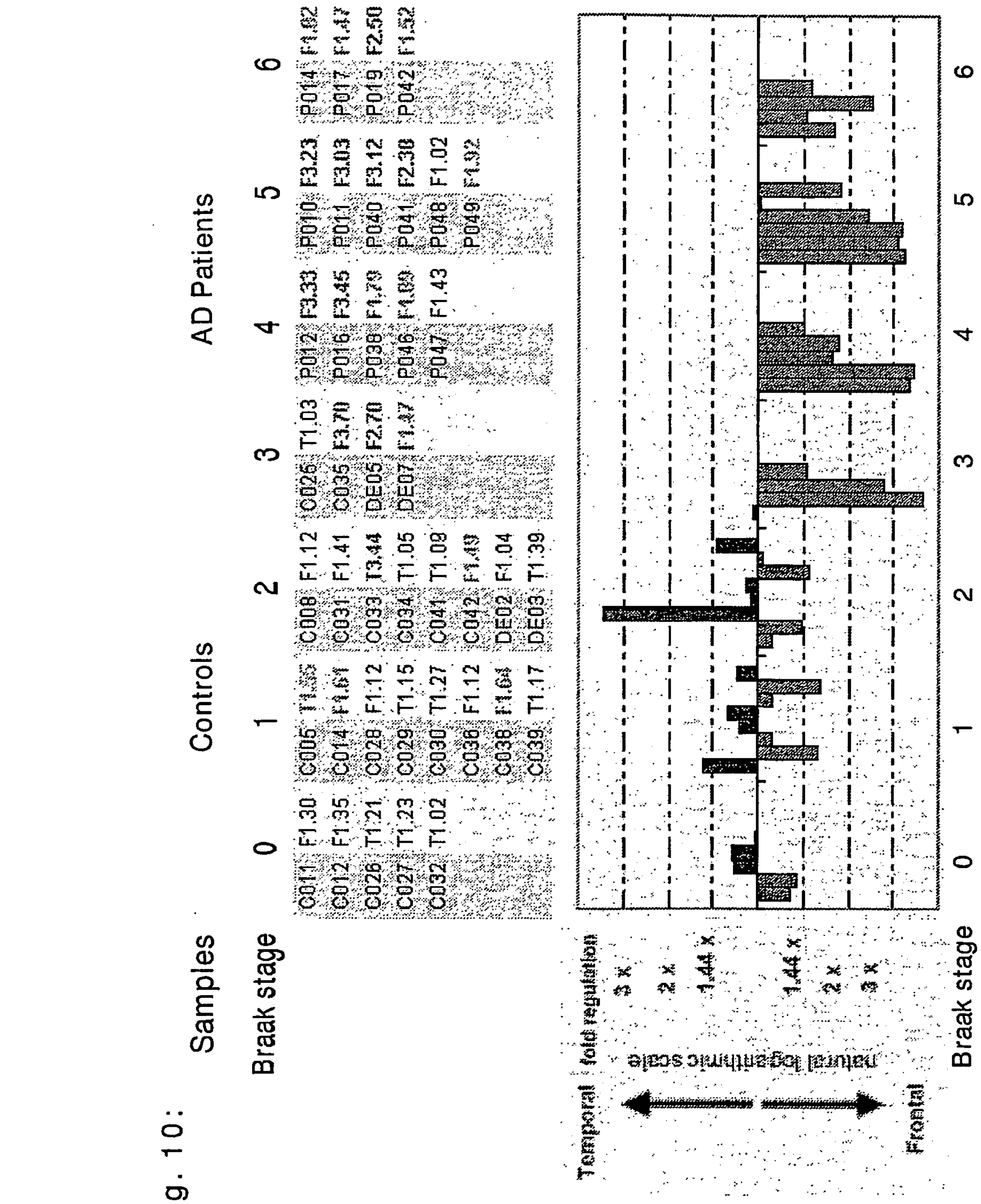
Samples
Braak stage
Controls
AD Patients
0
C011 F1.30
C012 F1.35
C026 T1.21
C027 T1.23
C032 T1.02
1
C005 T1.55
C014 F1.61
C028 F1.12
C029 T1.15
C030 T1.27
C036 F1.12
C038 F1.64
C039 T1.17
2
C008 F1.12
C031 F1.41
C033 T3.44
C034 T1.05
C041 T1.09
C042 F1.49
DE02 F1.04
DE03 T1.39
3
C025 T1.03
C035 F3.70
DE05 F2.70
DE07 F1.47
4
P012 F3.33
P016 F3.45
P038 F1.79
P046 F1.89
P047 F1.43
5
P010 F3.23
P011 F3.03
P040 F3.12
P041 F2.38
P048 F1.02
P049 F1.92
6
P014 F1.92
P017 F1.47
P019 F2.50
P042 F1.52
Temporal
Frontal
fold regulation
natural logarithmic scale
3 x
2 x
1.44 x
1.44 x
2 x
3 x
Braak stage
0
1
2
3
4
5
6

Fig. 11:

| sample | Δ (fold) (hippocampus/ frontal cortex) |
|---|---|
| control C005 | 0.84 |
| control C008 | 0.62 |
| control C004 | 1.00 |
| patient P012 | 1.13 |
| patient P016 | 0.12 |
| patient P010 | 2.04 |
| patient P011 | 1.74 |
| patient P014 | 1.24 |
| patient P019 | 0.53 |

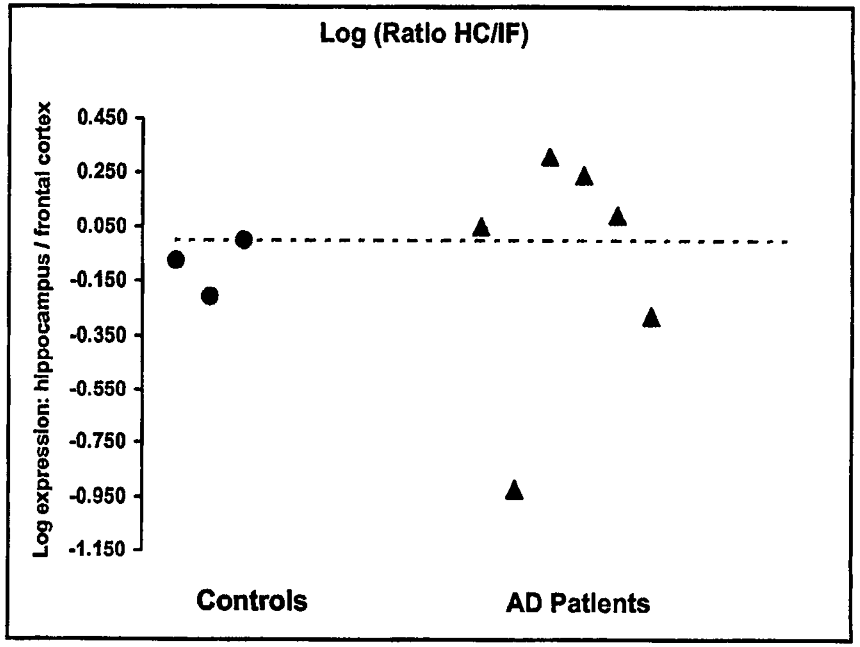

# Fig.12: Analysis of absolute mRNA expression of MAL2

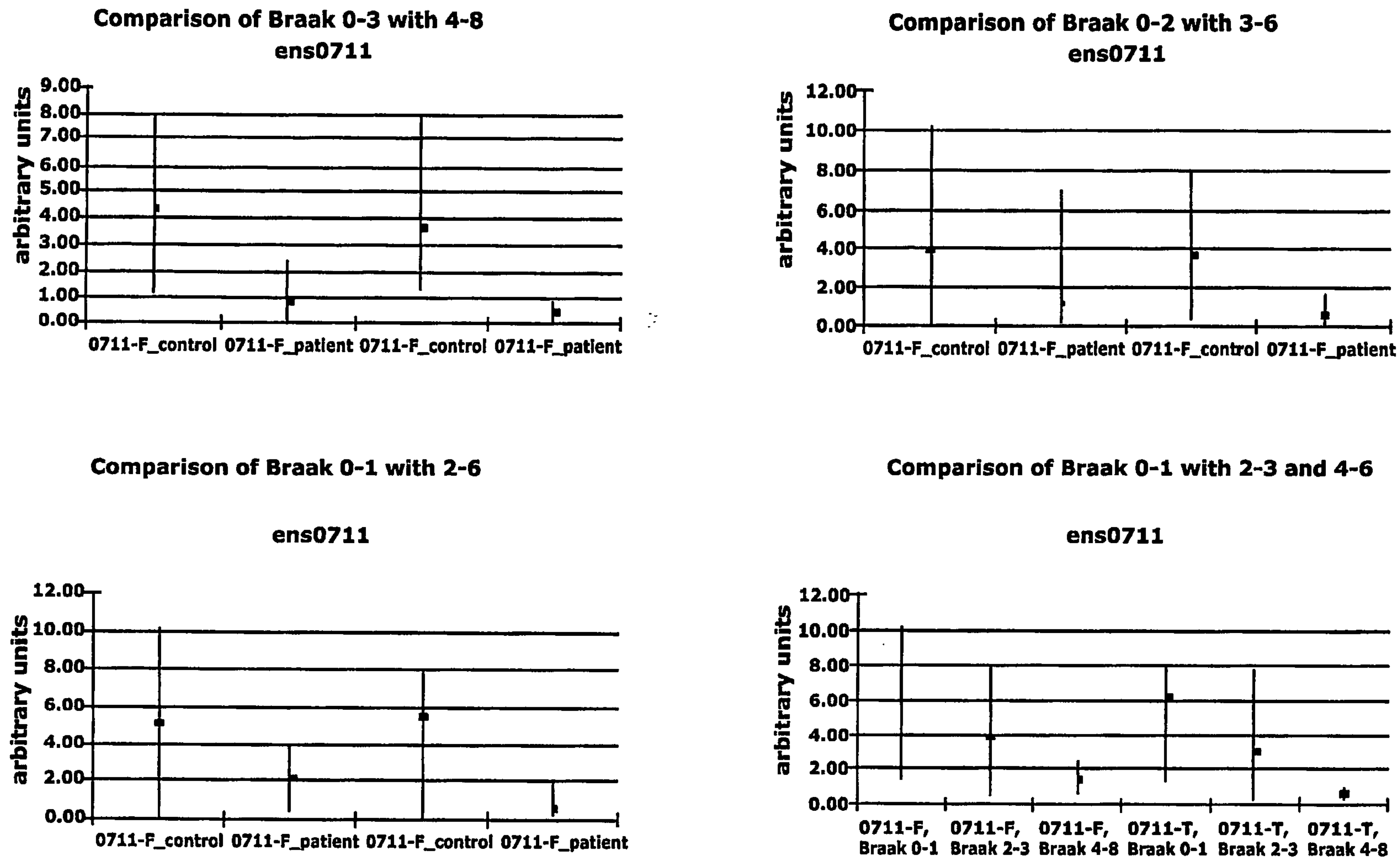

Fig. 13: Western Blot of H4APPsw cell protein extracts labeled with anti-MAL2-myc antibodies

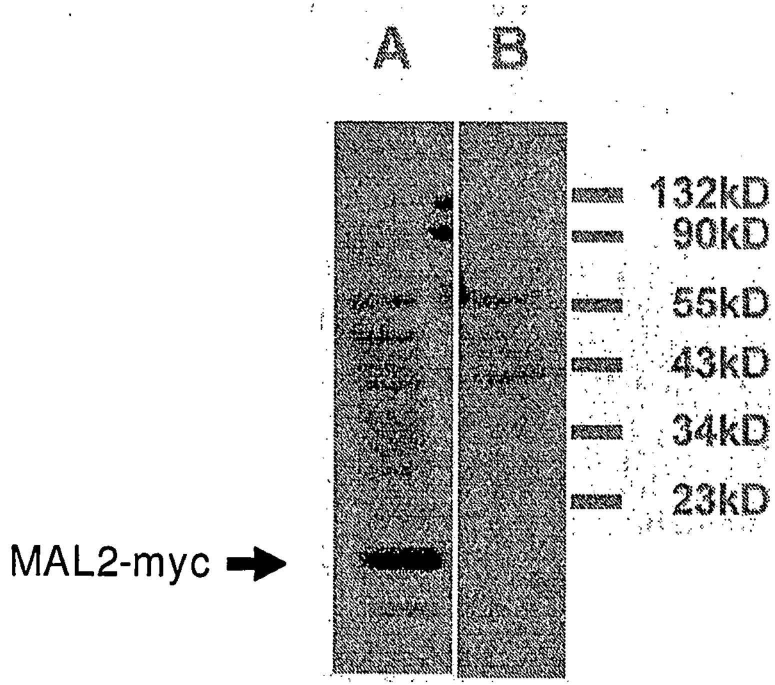

Fig. 14: Immunofluorescence analysis of MAL2 protein in neuroglioma cells

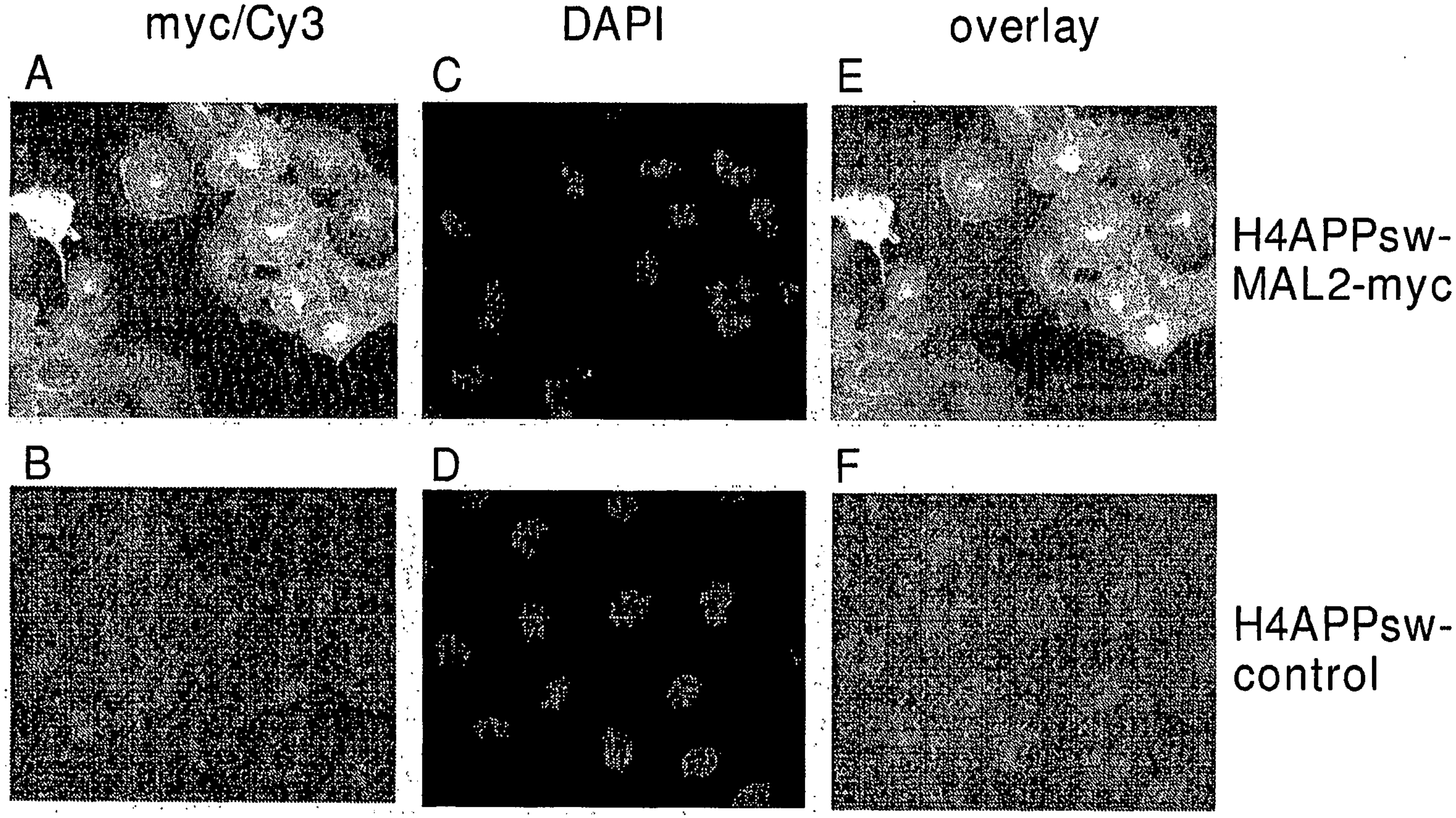

Fig. 15: Images of human pre-central brain sections labeled with anti-MAL2 antibodies and with DAPI

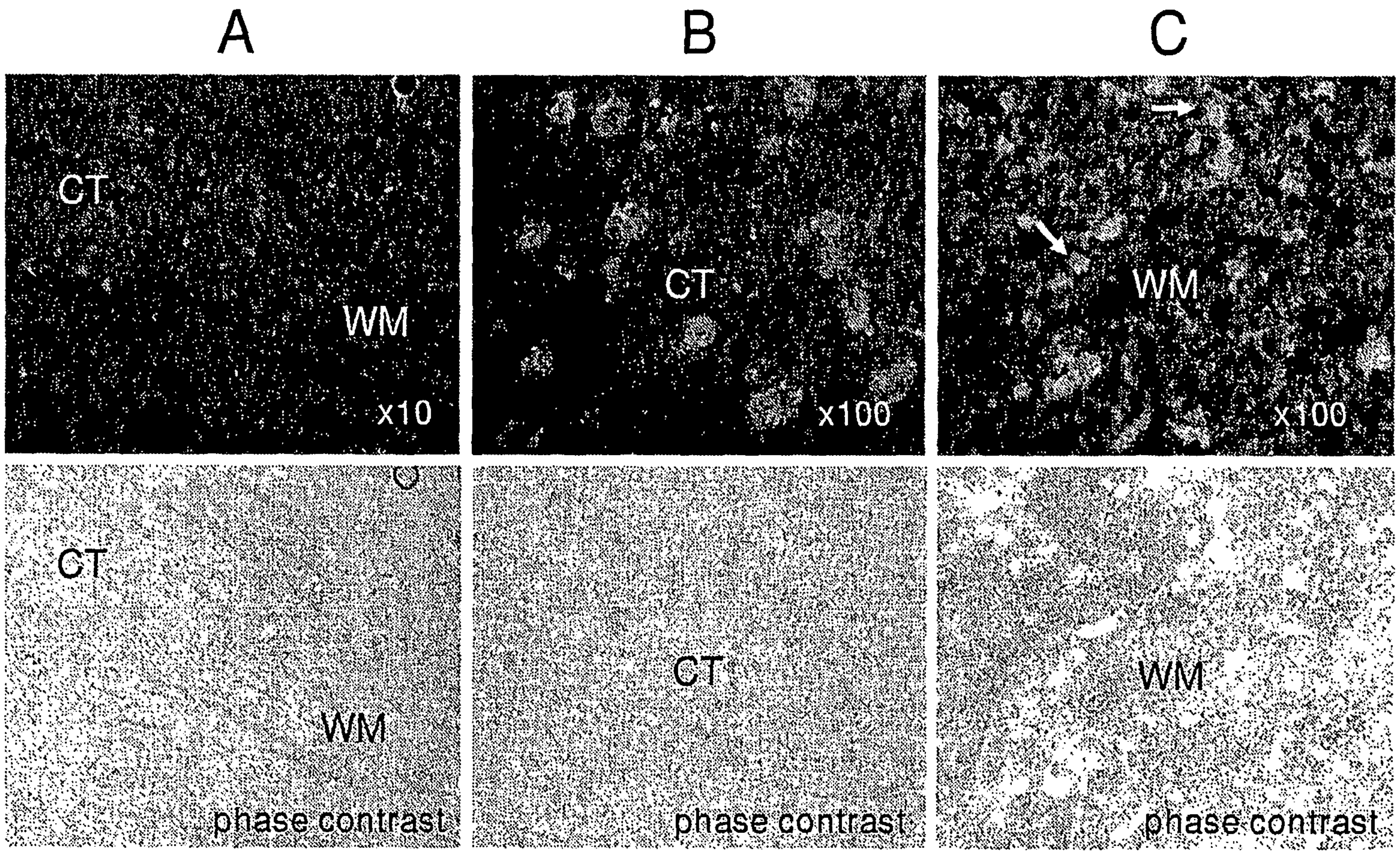

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **Vickers et al.** *Progress in Neurobiology,* 2000, vol. 60, 139-165
- **Selkoe.** *Physiological Rev,* 2001, vol. 81, 741-66
- **Greenfield et al.** *Frontiers Bioscience,* 2000, vol. 5, D72-83
- **Braak ; Braak.** *Acta Neuropathol,* 1991, vol. 82, 239-259
- **Schmitt et al.** *Neurology,* 2000, vol. 55, 370-376
- **Terry et al.** *Annals of Neurology,* 1981, vol. 10, 184-92
- **Strittmatter et al.** *Proc Natl Acad Sci USA,* 1993, vol. 90, 1977-81
- **Roses.** *Ann NY Acad Sci,* 1998, vol. 855, 738-43
- **Perez et al.** *Biochem Biophys Res Commun,* 1997, vol. 232, 618-621
- **Puertollano ; Alonso.** *Mol Biol Cell,* 1999, vol. 10, 3435-3447
- **Martin-Belmonte et al.** *J Biol Chem,* 2001, vol. 276, 49337-49342
- **Sanchez-Pulido et al.** *Trends Biochem Sci,* 2002, vol. 27, 599-601
- **Erne et al.** *J Neurochem,* 2002, vol. 82, 550-562
- **Frank et al.** *J Neurochem,* 2000, vol. 75, 1927-1939
- **Frank et al.** *J Neurochem,* 1999, vol. 73, 587-597
- **Wilson et al.** *Genomics,* 2001, vol. 76, 81-88
- **DeMarco et al.** *J Cell Biol,* 2002, vol. 159, 37-44
- **Magyar et al.** *Gene,* 1997, vol. 189, 269-275
- **Altschul et al.** *J. Mol. Biol.,* 1990, vol. 215, 403-410
- **Altschul et al.** *Nucleic Acids Res.,* 1997, vol. 25, 3389-3402
- **Devereux et al.** *Nucleic Acids Res.,* 1984, vol. 12, 387
- **Pearson ; Lipman.** *Proc. Natl. Acad. Sci. USA,* 1988, vol. 85, 2444-2448
- **Wilbur ; Lipman.** *SIAM J. Appl. Math.,* 1984, vol. 44, 557-567
- **Needleman ; Wunsch.** *J. Mol. Biol.,* 1970, vol. 48, 443-453
- **Iqbal ; Swaab ; Winblad ; Wisniewski.** Alzheimer's Disease and Related Disorders (Etiology, Pathogenesis and Therapeutics). Wiley & Sons, 1999
- **Scinto ; Daffner.** Early Diagnosis of Alzheimer's Disease. Humana Press, 2000
- **Mayeux ; Christemt.** Epidemiology of Alzheimer's Disease: From Gene to Prevention. Springer Press, 1999
- **Younkin ; Tanzi ; Christen.** Presenilins and Alzheimer's Disease. Springer Press, 1998
- **Braak ; Braak.** *Acta Neuropathology,* 1991, vol. 82, 239-259
- **Terry et al.** *Annals of Neurology,* 1981, vol. 10, 184-192
- **Sambrook ; Russell.** Molecular Cloning: A Laboratory Manual. Cold Spring Harbor Laboratory Press, 2001
- **Schena M.** Microarray Biochip Technology. Eaton Publishing, 2000
- **Harlow ; Lane.** Antibodies: A Laboratory Manual. Cold Spring Harbor Laboratory Press, 1999
- **Edwards R.** Immunodiagnostics: A Practical Approach. Oxford University Press, 1999
- **Behr.** *Chem Res,* 1993, vol. 26, 274-278
- **Mulligan.** *Science,* 1993, vol. 260, 926-931
- **Wolff.** *Curr Opin Neurobiol,* 1993, vol. 3, 743-748
- **Gillespie.** *DN&P,* 1992, vol. 5, 389-395
- **Agrawal ; Akhtar.** *Trends Biotechnol,* 1995, vol. 13, 197-199
- **Crooke.** *Biotechnology,* 1992, vol. 10, 882-6

- **Barinaga.** *Science,* 1993, vol. 262, 1512-1514
- **Wickstrom.** *Trends Biotechnol,* 1992, vol. 10, 281-287
- **Hannon.** *Nature,* 2002, vol. 418, 244-251
- **McCelland ; Pardee.** Expression Genetics: Accelerated and High-Throughput Methods. Eaton - Publishing, 1999
- **Capecchi.** *Science,* 1989, vol. 244, 1288-1292
- **Hogan et al.** Manipulating the Mouse Embryo: A Laboratory Manual. Cold Spring Harbor Laboratory Press, 1994
- **Jackson ; Abbott.** Mouse Genetics and Transgenics: A Practical Approach. Oxford University Press, 1999
- **Schwarz et al.** *Biochemistry,* 1999, vol. 38, 9456-9464
- **Krivosheev ; Usanov.** *Biochemistry-Moscow,* 1997, vol. 62, 1064-1073
- **Harlow et al.** Antibodies, A Laboratory Manual. Cold Spring Harbor Laboratory Press, 1988
- **Dubel ; Breitling.** Recombinant Antibodies. Wiley-Liss, 1999
- **Harlow ; Lane.** Using Antibodies: A Laboratory Manual. Cold Spring Harbor Laboratory Press, 1999
- **Edwards R.** Immunodlagnostics: A Practical Approach. Oxford University Press, 1999
- **Liang ; Pardee.** *Science,* 1995, vol. 267, 1186-7
- **von der Kammer et al.** *Nucleic Acids Research,* 1999, vol. 27, 2211-2218
- **Liang et al.** *Nucleic Acids Research,* 1994, vol. 22, 5763-5764
- **Zhao et al.** *Biotechniques,* 1995, vol. 18, 842-850